# EUROPEAN PATENT SPECIFICATION

(11) **EP 0 506 846 B1**
(45) Date of publication and mention of the grant of the patent: **19.07.1995**
(21) Application number: 91902443.0
(22) Date of filing: 20.12.1990
(51) Int. Cl.: C07K 1/10, C07K 1/12, G01N 33/68

(54) **AMINO ACID THIOHYDANTOIN METHOD AND REAGENT**
VERFAHREN UND REAGENS ZUR HERSTELLUNG DER AMINOSÄURE THIOHYDANTOIN
PROCEDE DE PRODUCTION DE THIOHYDANTOINE ET REACTIF UTILISE

(30) Priority: 21.12.1989 US 454666; 29.06.1990 US 547088
(43) Date of publication of application: 07.10.1992
(73) Proprietor: APPLIED BIOSYSTEMS, INC., Foster City California 94404 (US)
(72) Inventor: HAWKE, David, H., Hayward, CA 94544 (US); BOYD, Victoria, San Carlos, CA 94070 (US)
(74) Representative: West, Alan Harry
(86) International application number: PCT/US90/07567
(87) International publication number: WO 91/09868

(56) References cited:
- US-A- 4 837 165

## Description

The present invention relates to methods for forming an amino acid thiohydantoin, for determining the C-terminal amino acid of a peptide, for sequencing a peptide from its C-terminal peptide end, and to reagents useful in the methods.

### References

Cromwell, L.D., et al., Biochemistry 8:4735 (1969).

Edman, P., Acta Chem Scand, 4:277 (1950).

Hawke, D.H., et al, Anal Biochem, 166:298 (1987).

Kenner, G.W., et al., J Chem Soc, 673 (1953).

Meuth, J.L., et al., Biochemistry, 16:3750 (1982).

Miller, M.J., et al, J Org Chem, 42:1750 (1977).

Miller, C.G., et al, in "Techniques in Protein Chemistry (Hugh, T.E., ed), Academic Press, pp. 67-78 (1989).

Miller, C.G., et al., Abstract T188 from the Third Symposium of the Protein Society, Seattle WA (July 29-August 2, 1989).

Parham, M.E., et al, Biochem Biophys Res Commun, 80:7 (1978).

Rangarajan, M, in "Protein/Peptide Sequence Analysis: Current Methodologies" (Bhown, A.S., ed), CRC Press, pp 136-144 (1988).

Shively, J.E., et al, TIBS 14:246 (1989).

Stark, G.R., in Methods in Enzymology (Hirs, C.H.W., et al, eds.), Vol 25, p 369 Academic Press (1972).

Tarr, G.E., in "Methods in Protein Sequence Analysis," (Whittmann-Liebold, B., ed) Springer Verlag, pp. 129-151 (1988).

Woodward, R.B., et al., Tetrahedron, Supplement #7, 415-440 (1966).

Woodward, R.B., et al., J Am Chem Soc, 83:1007-1009 (1961).

Yamashita, S., Biochimica et Biophysica Acta, 229:301-209 (1971).

### Background of the Invention

Determining the amino acid sequence, i.e., primary structure, of a peptide is central to understanding the structure of the peptide, as well as to manipulating the peptide to achieve desired properties in a modified or altered form. In addition, the amino acid sequence of a peptide is useful in a variety of recombinant DNA procedures for identifying the gene coding sequence of the peptide, for producing the peptide recombinantly, and/or for producing site-specific modifications of the peptide.

Methods for use in N-terminal sequencing are well known (e.g., Edman). Despite the relative ease and reliability of N-terminal sequencing methods, it is often desired to obtain C-terminal amino acid sequence information which may be inaccessible or only obtained with difficulty by this method. Information about the carboxy terminal sequence may be useful for certain types of recombinant DNA procedures, particularly since the C-terminal end of the coding region of a protein corresponds to the end closest to a poly A tail, which is likely to be present in cDNA clones.

Three general approaches have been proposed for C-terminal peptide sequencing: enzymatic, physical, and chemical. These methods and their inherent limitations have been summarized in the above-cited parent application. Briefly, neither enzymatic not physical determinations have proven satisfactory to date. In view of this, considerable effort has been invested in developing chemical methods for determining C-terminal amino acids residues, and for C-terminal sequencing. An inherent difficulty in C-terminal sequencing is the relatively poor reactivity of the carboxyl group, in contrast to the relative ease of addition at the N-terminal amino group. Of the reaction methods which have been proposed for C-terminal sequencing, three have received special attention.

The first method involves generating a carboxyamido derivative at the C-terminal end of the peptide, followed by reaction with bis(*I,I*-trifluoroacetoxy)iodobenzene, to form a derivative which rearranges and hydrolyses to a shortened carboxyamidopeptide and the aldehyde derivative of the C-terminal amino acid (Parham). The method has been successfully carried out only to 3-6 cycles before the reaction halts. In a second, related approach, the carboxy terminus is reacted with pivaloylhydroxamate to effect a Lossen rearrangement. One limitation of the method is that the chemistry does not degrade aspartic and glutamic acid residues (Miller, 1977).

The most widely studied of the C-terminal chemistries is the thiohydantoin (TH) reaction. In one general method for carrying out the TH method, the carboxyl group is activated with an anhydride, such as acetic anhydride, in the presence of an isothiocyanate (ITC) salt or acid, to form a C-terminal peptidyl-TH via a C-terminal ITC intermediate (Stark, 1972). The peptidyl-TH can be cleaved to produce a shortened peptide and a C-terminal amino acid TH, which can be identified, e.g., by high pressure liquid chromatography (HPLC). The coupling conditions in this method typically require about 90 minutes at a 60-70°C (Meuth), and often lead to degradation of some of the amino acid side chains in the peptide. Further, the anhydride reagent is relatively unstable, and therefore presents storage problems.

A C-terminal TH sequencing method which can be carried out under milder conditions has been described by one of the inventors and co-workers (Hawke). Using trimethylsilyl isothiocyanate (TMSITC) as the reagent, TH formation was achieved by activation of the peptide with acetic anhydride for 15 min at 50°C, followed by reaction with TMS-ITC for an additional 30 min at 50°C. The method suffers from the disadvantage, noted above, of peptide exposure to a highly reactive anhydride activating agent. In addition, and like the related TH-generating methods described above, the TH-amino acid reaction products are racemized, and thus the method cannot be used to distinguish D- and L-form amino acids.

The C-terminal sequencing methods involving TH formation just described commonly lead to racemized products. A modification of the C-terminal reaction employing phosphoryl isothiocyanatidate reagent has been proposed (Kenner). Although TH was produced, the reaction was too slow to be very useful. Miller et al have proposed a related method, but using a mercapto-benzothiazole derivative. The rationale for using this compound is that cyclization could occur with concomitant opening of the thiazole ring.

### Summary of the Invention

It is one general object of the invention to provide a C-terminal peptide sequencing method which involves relatively rapid C-terminal peptidyl TH formation under relatively mild reaction conditions, and in particular, conditions which do not involve the use of anhydride activation reagents.

It is another object of the invention to provide a novel reagent for use in C-terminal sequencing.

It is still another object of the present invention to provide a C-terminal sequencing method which can be carried out under conditions in which at least some of the reactants of the sequencing reaction are sequestered on a solid support.

It is a more general object of the invention to provide a method and immobilized reagent for producing an amino acid TH.

The invention includes, in one aspect, a method for producing a thiohydantoin (TH) of a protected amino acid having a free carboxylic acid group. The protected amino acid is reacted with a mixed anhydride of isothiocyanic acid and a carboxyl, carbonic or sulfonic acid, under conditions in which the carboxylic acid group of the amino acid is deprotonated. In a related aspect, the invention includes a method for determining the C-terminal amino acid residue of a peptide. The peptide is reacted with a mixed anhydride of isothiocyanic acid and a carboxyl, carbonic or sulfonic acid, under conditions in which the carboxylic acid group of the C-terminal amino acid is deprotonated, to produce the corresponding C-terminal peptidyl thiohydantoin (TH). C-terminal hydrolysis of the peptidyl-TH bond releases the amino acid TH, which can then be identified, e.g., by HPLC, as an amino acid TH adduct.

In one embodiment the mixed anhydride reagent used in the method has the form: 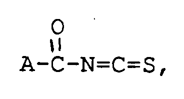
where A is an alkyl, aryl, or aryloxy group.

In one general embodiment, the N-protected amino acid or peptide is reacted with a solution-phase mixed anhydride reagent.

In another general embodiment of the invention, the N-protected amino acid or peptide is contacted with a solid support derivatized with a mixed anhydride of isothiocyanic acid and carboxylic, carbonic or sulfonic acid, under basic conditions in which the carboxyl group of the amino acid is deprotonated. The amino acid TH formed in solution is separated from the solid support, such as by eluting the product from the support.

The acyl ITC support preferably has the form: 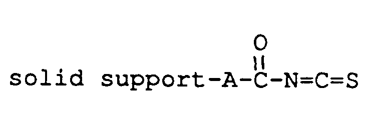
where A is an alkyl, alkoxy, aryl, or aryloxy group attached to the solid support. The solid support may be derivatized by activation with Woodwards Reagent K or an anhydride reagent, followed in both cases by reaction with an ITC, or by reaction with a solution-phase acyl ITC of the type disclosed herein.

For use in C-terminal peptide sequencing, the method of the invention further includes cleaving the C-terminal amino acid TH from the residual peptide and identifying the cleaved amino acid TH, such as by HPLC. In the embodiment employing a solid-phase reagent, the peptidyl TH is preferably contacted with a second solid support having a cleaving agent effective to cleave the peptidyl TH from the residual peptide. The peptide is preferably retained on the solid support, either by ion-exchange binding, or by covalent linkage which forms during the cleavage reaction. After elution of the amino acid TH in the solution phase, the residual peptide may be released and recycled for further sequence determination.

In another aspect, the invention includes an activated support composed of a solid support derivatized with a mixed anhydride of isothiocyanic acid and carboxylic or carbonic acid, and preferably having the form: 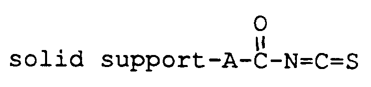
where A is an alkyl, alkoxy, aryl, or aryloxy group attached to the solid support.

Also forming part of the invention is a system for solution-phase C-terminal sequencing. The system includes an activated support of the type just described, and preferably a second solid support capable of (i) cleaving a peptidyl TH to release the amino acid TH, and (ii) binding the residual peptide to the support. The residual peptide can be selectively released from the support after the amino acid TH has been recovered. The system may include a series of such supports, for successive C-terminal peptide determinations.

These and other objects and features of the invention will become more fully apparent when the following detailed description of the invention is read in conjunction with the accompanying drawings.

### Brief Description of the Drawings

Figure 1 shows mixed anhydrides of isothiocyanic acid (compounds I-V) which may be used in practicing the present invention;
Figure 2 shows the proposed mechanism of reaction of a peptide carboxyl group with an alkyl acyl ITC reagent, in accordance with the invention, to form a peptidyl ITC compound;
Figure 3 shows a proposed mechanism of formation of a peptidyl thiohydantoin (TH) and hydrolysis of the terminal amino acid TH to form an amino acid TH and a shortened peptide;
Figure 4A and 4B are HPLC chromatograms showing the comparative yields of C-terminal amino acid-TH formed in a method which uses TMS-ITC in acetic anhydride, according to prior art methods (4A), and a benzoyl-ITC mixed anhydride reagent, according to the present invention (4B);
Figure 5A and 5B show HPLC chromatograms of amino acid-TH compounds formed for C-terminal leucine (L), and C-terminal methionine (M), respectively;
Figure 6A and 6B show HPLC chromatograms of the first and second cycles, respectively, in the C-terminal sequencing of the pentapeptides leucine enkephalin (TGGFL);
Figure 7A and 7B show HPLC chromatograms of Ile-TH formed by reaction of Ile with TMS-ITC in acetic anhydride, according to prior art methods (7A), and by reaction with benzoyl-ITC mixed anhydride reaction, according to the present method (7B);
Figure 8 illustrates the use of the method of the invention for C-terminal peptide sequencing;
Figure 9 shows the synthesis of a mercaptobenzothiazole compound which may be used for generating C-terminal amino acid-phenyl TH derivatives;
Figure 10 illustrates the proposed reaction or a mercaptobenzothiazole compound with a C-terminal amino acid, and subsequent cleavage to form the amino acid-phenylTH compound;
Figure 11 shows the reaction steps for forming an activated solid support using Woodwards Reagent K to activate carboxyl groups on the support;
Figure 12 shows the reaction steps for forming a solid support using an acid anhydride to activate the carboxyl groups on the support;
Figure 13 shows the reaction steps for forming an activated solid support using a solution-phase acyl-ITC to activate carboxyl groups on the support;
Figure 14 shows the reaction steps for forming an activated solid support derivatized with a mixed anhydride of carbonic acid and ITC;
Figures 15A and 15B show reaction schemes for formation of a peptidyl TH by peptide reaction with an activated acyl ITC support, in accordance with the invention (15A), and the cyclization of peptidyl-ITC to form peptidyl TH (15B);
Figure 16 shows a variety of mixed anhydride ITC compounds, with the calculated atomic charge on the carbonyl carbon, sulfonyl sulfur atom, and ITC carbon atom indicated for each compound;
Figure 17 illustrates the steps in cleaving a peptidyl TH by a cation-exchange resin, and binding of the residual peptide to the resin support;
Figure 18 illustrates the steps in cleaving a peptidyl TH by a solid support derivatized with hydroxylamine groups, and subsequent hydrolytic release of the residual peptide from the resin support; and
Figure 19 illustrates the steps in a C-terminal sequencing operation in accordance with the invention.

### Detailed Description of the Invention

### I. Solution-Phase Method and Reagents

In one general embodiment of the invention, formation of an amino acid TH is carried out by reacting an N-protected amino acid or peptide with a solution-phase mixed anhydride reagent. That is, the mixed anhydride of isothiocyanic acid and a carboxyl, carbonic or sulfonic acid is in a free (non-immobilized) form. The N-protected amino acid or peptide may be in solution or immobilized on a solid support, as described below. The mixed anhydride reagents used in this method are referred to herein as solution-phase reagents, to distinguish the reagents from the solid-phase mixed anhydride reagents described in Section II below. Similarly, the general method in which an N-protected amino acid or peptide is reacted a solution-phase reagent is referred to as a solution-phase method. It will be appreciated that the "solution-phase method" may include a solid-phase support on which an amino acid, or more typically, a peptide is immobilized.

### IA. Mixed Anhydride Reagent

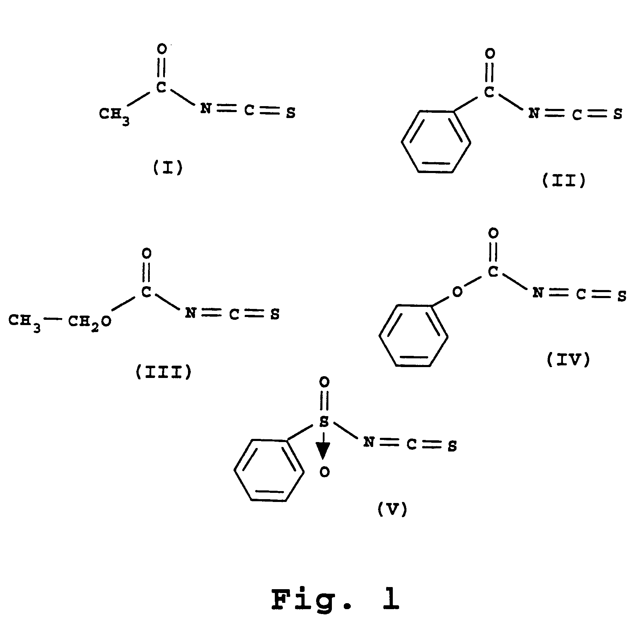

The reagent employed in the method of the present invention is a mixed anhydride of isothiocyanic acid (ITC) and carboxylic, carbonic, or sulfonic acid. Several exemplary mixed anhydride reagents are illustrated in Figure 1. These include:
(a) an alkyl or aryl acyl-ITC compound, as exemplified by acetyl ITC (I), and benzoyl ITC (II). The alkyl group may be selected from the group of alkyl and cycloalkyl compounds, such as methyl, ethyl, propyl, t-butyl, and related hydrocarbon compounds, including unsaturated compounds, linked to the acyl carbon through a carbon-carbon linkage. The aryl group may be a benzene, substituted benzene, or related compound linked to the acyl carbon through an aryl ring carbon atom. These two classes of compounds are also referred to herein as mixed anhydrides of ITC and carboxylic acids, since hydrolysis of the CO-N bond yields a carboxylic acid and HNCS;
(b) an alkoxy or aryloxy carbonyl-ITC compound, as exemplified by ethoxy acyl-ITC (III) or benzoxy carbonyl-ITC (IV). The ether linked alkyl or aryl groups in the compounds are as described in (a). These two classes of compounds are also referred to herein as mixed anhydrides of ITC and carbonic acids, since hydrolysis of the CO-N bond yields a carbonic acid and HNCS; and
(c) an alkyl or aryl sulfonyl-ITC compound, as exemplified by benzylsulfonyl-ITC (V). The alkyl or aryl groups in the compounds are as described in (a). These two classes of compounds are also referred to herein as mixed anhydrides of ITC and sulfonic acids, since hydrolysis of the CO-N bond yields a sulfonic acid and HNCS;
   In one preferred embodiment of the invention, the solution-phase reagent is a mixed anhydride of carboxylic acid and ITC, as described in (a), and has the general formula: 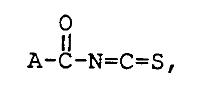 where A may be any carbon-containing group, such as an alkyl or aryl group, which allows reagent solubility in the non-aqueous solvent used in the TH-forming reaction, and reactivity toward a peptide acyl group, in the isothiocyanation reaction to be described.

Some compounds in this class may be obtained commercially, or prepared according to known procedures. One method of preparing acyl-substituted ITCs involves dissolving an isothiocyamate in a dry, inert solvent containing a base, and slowly adding the desired acyl chloride. For example, a synthesis of benzoyl ITC employs benzoyl chloride in acetonitrile or dichlormethane or toluene as an inert solvent, and diisopropyl ethyl amine as the base.

Mixed anhydrides of ITC and carbonic acid (ROC(O)NCS) may be formed by analogous methods, using a selected alkyl or aryl ester chloride in reaction with the ITC salt. Similarly, mixed anhydrides of ITC and sulfonic acid are formed by reacting the desired benzenesulfonyl chloride with an ITC, as outlined in Example 7.

### IB. Solution-Phase Reaction Method

In practicing the method of the invention, an N-protected amino acid or the peptide whose C-terminal amino acid is to identified is reacted with the mixed anhydride reagent under basic conditions in which the carboxyl group of the C-terminal amino acid is deprotonated. The C-terminal amino acid is converted to a C-terminal amino acid TH which is linked to the penultimate C-terminal amino acid (where the reaction is with a peptide) in the peptide through a ring-nitrogen amide bond.

As used above, the term "peptide" is intended to include both peptides and proteins, these generally being distinguished by less than or more than 100 amino acids, respectively, and N-protected amino acids. The method will be described particularly for use in determining a C-terminal amino acid of a peptide. However, it will be understood that the method is also applicable to generating amino acid thiohydantoins from free, N-protected amino acids.

Where the method is used to identify only the C-terminal amino acid residue of the peptide, the peptide may be reacted in free form, i.e., unattached to a solid support, where the amino terminus is protected. Typically, where successive rounds of C-terminal amino acid identification are desired, for purposes of C-terminal sequencing, the peptide is first attached at its N-terminus or internal side chain to a solid support.

Methods for attaching peptides to solid supports, such as activated glass beads, carboxylated or aminated resin beads, and the like are well known. In one preferred approach, employed in the method described in Example 1, the dipeptide Leu-Val was immobilized to aminated resin by a benzyl diisothiocyanate.

The peptide, either in free or immobilized form, is dissolved or suspended in a suitable solvent under basic, preferably non-aqueous conditions effective to deprotonate the C-terminal carboxylic acid group. The solvent preferably also includes pyridine, which may serve an important catalytic role in thiohydantoin (TH) ring formation, as discussed below. One preferred solvent is anhydrous acetonitrile containing 10% anhydrous pyridine. A typically reaction volume is about 100 »L reaction solvent per 1-3 mg peptide.

The mixed anhydride reagent from above is now added to the peptide suspension, preferably in molar excess of the peptide carboxyl group available for reaction in the reaction suspension. Typically, about 10 »L of the reagent are added per 100 »L reaction solvent.

The TH-forming reaction is preferably carried out at 50-70°C, for about 10-60 minutes, preferably at about 50°C for 15-30 minutes, to minimize reaction of the ITC reagent with non-carboxyl groups in the peptide. Following this, the reaction mixture is cooled to room temperature, and the peptidyl TH is recovered. Where the peptide is attached to a solid support, the support recovery is accomplished readily by washing the support, for example, with several volumes of acetonitrile, and drying the washed support, for example, by vacuum centrifugation. Where the peptide is reacted in free form, the peptidyl TH may be recovered by chromatographic separation or the like in a solvent system which does not produce end-terminal cleavage of the amino acid TH from the peptide.

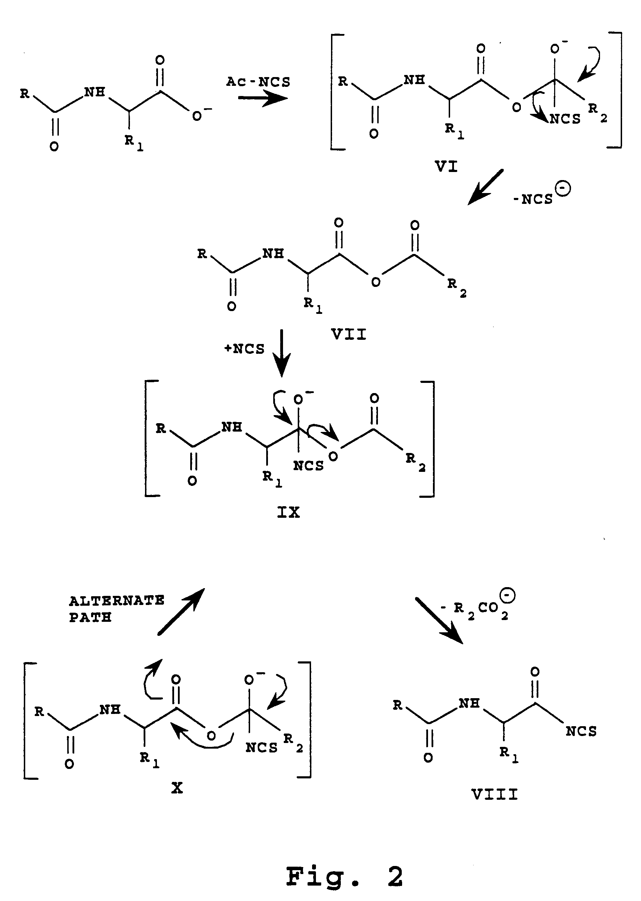

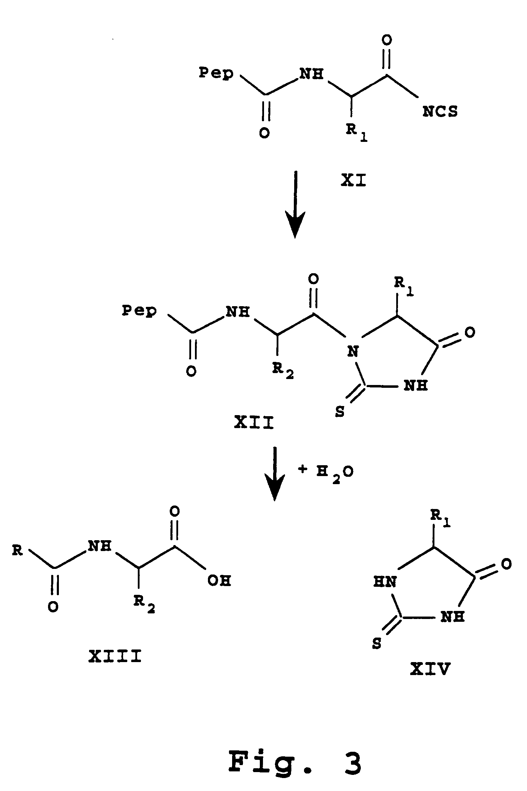

Figures 2 and 3 show the proposed mechanism of the reaction for peptidyl TH formation in accordance with the method of the invention. In the reaction mechanism shown at the top in Figure 2, the mixed anhydride reagent reacts through an unstable ITC intermediate VI to form the peptidyl mixed anhydride shown at VII in the figure. The anhydride then reacts with a free thiocyanate ion produced in the anhydride reaction, to form the peptidyl ITC compound shown at VIII in the figure, via the tetrahedral intermediate IX. In this proposed mechanism, the mixed anhydride reagent functions both as an activating reagent, to form a reactive peptidyl anhydride, and as a source of thiocyanate ions for reaction with the anhydride.

An alternative reaction mechanism is shown in the second line in Figure 2. Here the thiocyanate group in the tetrahedral intermediate X in the figure migrates to the peptide carbonyl carbon, forming the tetrahedral intermediate IX, which rapidly collapses to form the peptidyl ITC compound shown at VIII.

With continued reference to Figure 2, there are two possible electrophilic sites of reaction of the peptidyl deprotonated (nucleophilic) oxygen atom. The first site, and the one shown in the Figure 2 reaction scheme, is the carbonyl carbon. The second site is the thiocarbonyl carbon, which would lead to the wrong reaction products. Experiments conducted in support of the present invention indicate that more electrophilic substitution at the carbonyl carbon favors reaction at the thiocarbonyl carbon, based on the percentage of the amino acid-TH compound formed. Thus, for example, alkyl and aryl carbonyl groups (the mixed anhydrides of isothiocyanic acid and carboxylic acids) give the highest percentage of the desired TH product, and the more electro-negative ester reagents (mixed anhydrides of isothiocyanic acid and carbonic acid), a lower percentage of the desired product. Under present conditions, the sulfonic acid mixed anhydride reagent gives the lowest percentage of the desired TH product.

The reaction of the peptidyl ITC compound XI to form the desired peptidyl TH is shown in Figure 3, and presumably involves an electrophilic attack by the thiocyanate carbon on the amide nitrogen in the peptide ITC compound, with rapid cyclization to form the peptidyl TH shown at XII in the figure. The cyclization reaction may be catalyzed by pyridine, suggesting that pyridine may be reacting with the thiocyanate moiety to enhance the reactivity of the reactive carbon center, as has been proposed (Miller 1988).

Specific reaction conditions for forming an amino acid or peptidyl TH, in accordance with the present invention are given in Examples 1A, 2, 3, and 6 for benzoyl-ITC; in Example 4, for trimethylacetyl-ITC; in Example 5, for ethoxycarbonyl; and in Example 7B, for benzenesulfonyl-ITC.

### IC. Formation and Identification of Amino Acyl Hydantoin

This section describes the final steps of the method of the invention, which include (a) treating the peptidyl TH under cleavage conditions effective to cleave the amide linkage joining the TH to the peptide, (b) isolating the amino acyl TH released by the cleavage reaction, and (c) identifying the isolated amino acyl TH.

A variety of cleavage reaction conditions are known. Hydrolytic cleavage with 12 N HCl, dilute alkali (Kenner), or saturated aqueous triethylamine have been reported. These cleavage reactions are reported to yield up to 70% percent cleavage, but the extreme pH conditions can lead to ring opening and/or damage to internal peptide side chains. More recently, cleavage by treatment with acetohydroxamate in pyridine at pH 8.0 was reported (Meuth). The method affords recovery yields of up to 60-80% of the C-terminal TH (Miller, 1988). A related method involves treatment with primary or secondary amines in acetonitrile.

The cleavage reactions are typically carried out at room temperature or higher, for 15-60 minutes, depending on the cleavage agent used. The course of the reaction can be readily followed, for purposes of optimizing TH release, and minimizing ring opening and damage to the shortened peptide, by examining the integrity of the released amino acyl TH and/or shortened peptide, according to analytical methods described below, during the course of the reaction.

In one preferred cleavage method described in Examples 1-4, the peptidyl TH is treated with 10% propylamine in acetonitrile at room temperature for 15 minutes. In a related method, described in Example 5, the peptidyl-TH was cleaved with tetra-N-butyl ammonium hydroxide in water containing 1 mg/ml dithiothreitol (DTT).

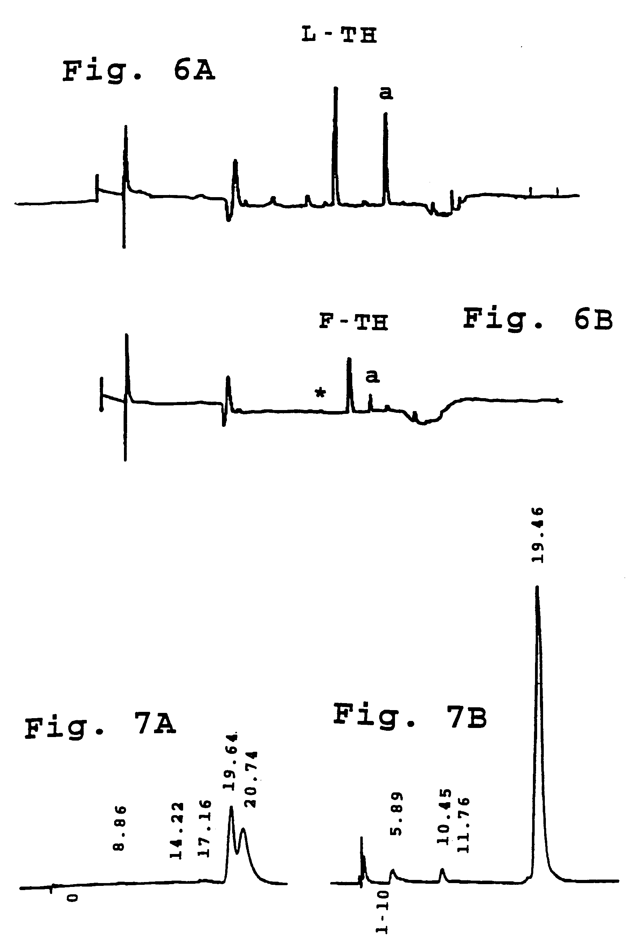

According to one aspect of the invention, it has been discovered that the TH-forming method, when carried out under acidic conditions, preserves the stereoisomeric form of C-terminal amino acid. This is illustrated in Example 6, where t-BOC protected Ile is deprotected with trifluoroacetic acid (TFA), in 25% H20, to yield a single L-form Ile-TH compound, as seen in Figure 7B. By contrast, reaction formation of deprotected Ile-TH by reaction with trimethylsilyl isothiocyanate (TMS-ITC), and deprotection (cleavage) with 12N HCl, in accordance with prior art C-terminal analysis methods (Hawke), produced the two diastereoisomeric forms seen as a doublet in FIgure 7A.

The results of the cleavage reaction are illustrated at the bottom in Figure 3. In the Figure 3 reaction, the cleavage produces a peptide shortened by one amino acid (compound XIII) and an amino acid TH (XIV) whose R₁ group is, of course, the amino acid side chain of the original C-terminal amino acid.

The amino acyl TH released from the peptide or the shortened peptide, or both, are analyzed to identify the N-terminal amino acid. Where the peptide is coupled to a solid support, the released TH can be easily separated by removing the cleavage-reaction solvent, e.g., by vacuum centrifugation, and extracting the released TH in a suitable solvent such as acetonitrile, as detailed in Example 1. Where the peptide is free in the cleavage reaction mixture, the mixture may be separated, e.g., by HPLC, as part of the method for identifying the compound.

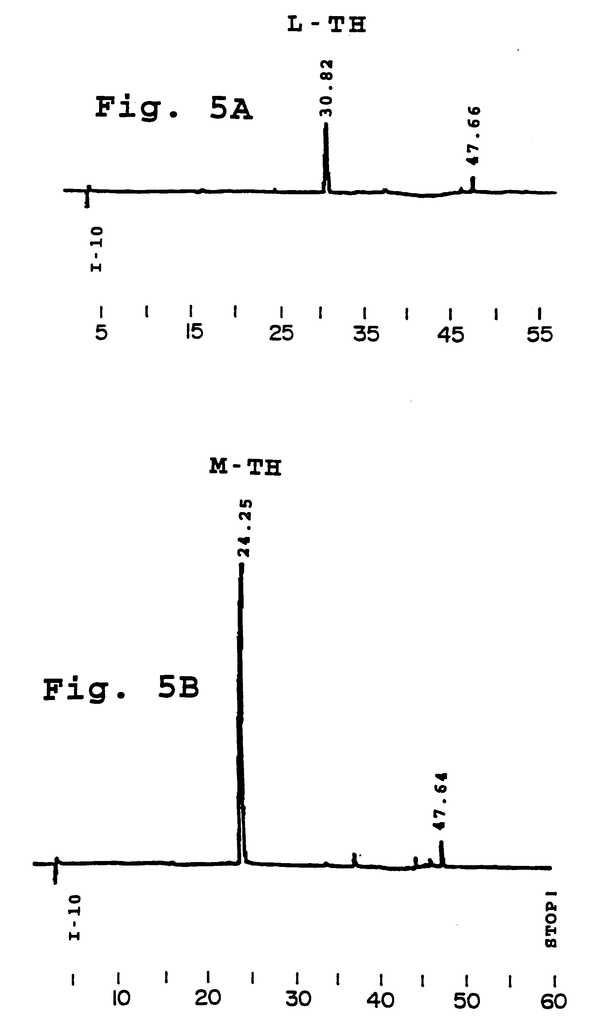

The released amino acid TH compound may be identified by known chromatographic methods, such as high pressure liquid chromatography (HPLC), according to standard procedures. Compound identification can be made conveniently by comparing the run times in the columns with the run times of known reference amino acid THs, prepared according to standard methods. The HPLC methods detailed in Example 1 are suitable. Figures 5A and 5B show HPLC profiles of leucine TH (L-TH) and methionine-TH (M-TH), and Figures 6A and 6B, or L-TH and phenylalanine-TH (F-TH).

Alternatively, the released and isolated amino acid TH can be identified by other available tools, such as mass spectrometry or NMR.

### ID. C-Terminal Amino Acid Sequencing

The method described in Section IC above is designed for determining the C-terminal amino acid residue of a peptide. It will be appreciated that repeated application of the method can be used for C-terminal amino acid sequencing of the peptide.

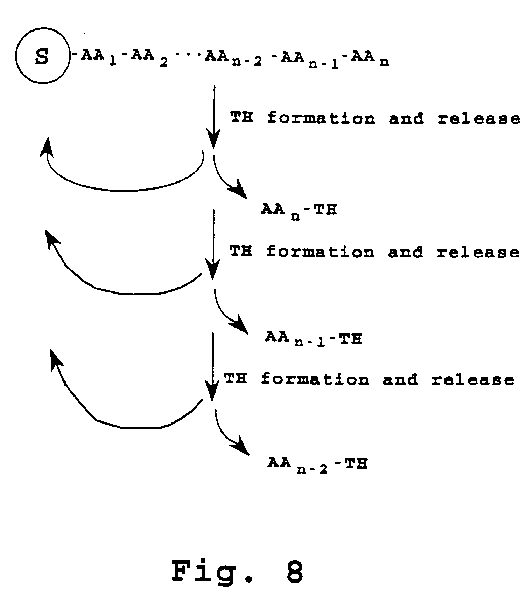

Figure 8 illustrates the sequencing method. The peptide to be sequenced is coupled to a solid support S, as indicated. The peptide is then carried through a first round of steps to (a) produce the C-terminal peptidyl TH, (b) cleave the terminal TH, yielding the C-terminal amino acyl TH (AAₙ) and a shortened peptide whose C-terminal residue is now AAₙ₋₁, and (c) identify the released amino acyl TH.

After washing the peptide support, the shortened peptide is treated with a second round of the above steps to release the next-in amino acid as an amino acid TH (AAₙ₋₁) and yield a peptide shortened by two C-terminal residues. This procedure is repeated until the peptide has been sequenced or until loss of resolution, due to incomplete TH formation and release, makes further sequencing impossible. The sequencing method is illustrated in Example 5, for the determination of the two C-terminal amino acid residues of leucine enkephalin (YGGFL). The HPLC chromatogram from the first round of analysis, given in Figure 6A, shows the C-terminal L-TH peak. The chromatogram from the second round, given in Figure 6B, shows the expected F-TH.

The above-detailed technique is easily automated using technology known for the automated sequencing of the N-terminal residues of peptides. One embodiment of a device to automatically sequence a peptide from the C-terminal end employs a solid support contained in a reaction vessel to which fresh solvent and reagents are added, and from which reaction mixtures and solvent washes are removed. The released amino acid TH's are isolated from the stored material for later identification by HPLC.

### IE. Alternative Mixed Anhydride Reagents

Although the present invention encompasses the mixed anhydrides of carboxylic, carbonic, and sulfonic acid described in Section IA, additional mixed anhydrides as disclosed below may be suitable for forming C-terminal amino acid-TH compounds.

One of the alternative reagents is a carbamate mixed anhydride of isothiocyanic acid, having the general formula: 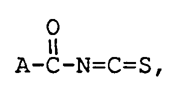
where A may be any amine, and preferably a quaternary amine, such as trimethyl amine. This reagent would be expected to have about the same electronegativity as the carbonate mixed anhydrides, and thus should provide a reasonable yield of the desired peptidyl-TH product.

Another alternative mixed anhydride is a benzoylmercapto-benzothiazole (MBT) XV, such as illustrated at the right in Figure 9. This compound may be prepared according to the synthetic method described in Example 8. Briefly, MBT XVI is dissolved in a dry, inert solvent in the presence of a suitable base, such as diisopropyl ethyl amine. A selected acyl chloride, e.g., benzoyl chloride XVII, is then slowly added at a reduced temperature to form the desired product. Related acyl MBT compounds, such as acetyl MBT, can be prepared by a similar method, using the desired acyl chloride starting material.

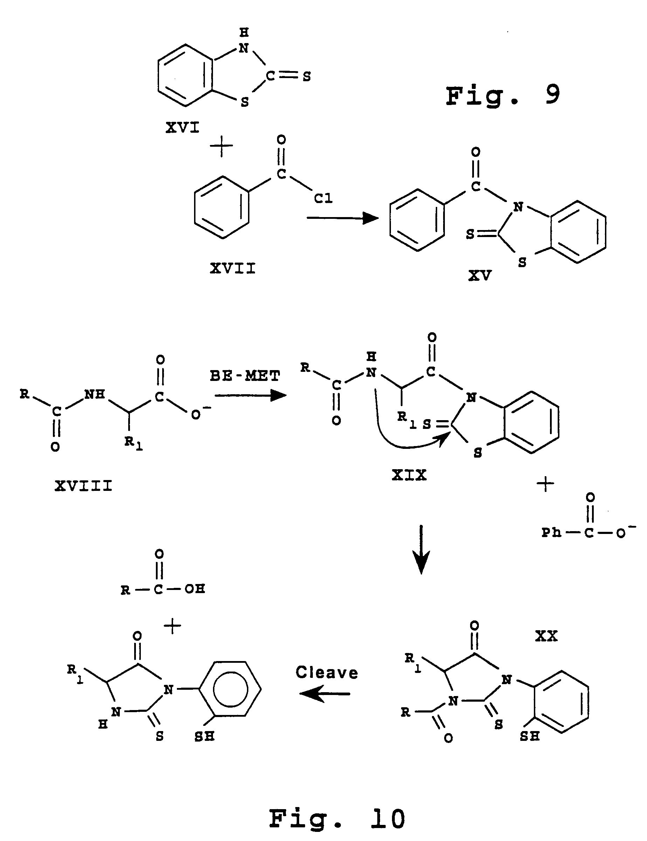

Figure 10 shows the proposed reaction steps in the formation of a peptidyl phenyl TH, employing an MBT reagent. The reaction conditions may be substantially similar to those described in Section B, with suitable adjustment in reaction time, if necessary, to ensure completion of the TH-formation reaction. Typical reaction conditions are given in Example 8.

With reference to Figure 10, the deprotonated peptide (XVIII) is reacted with benzoyl-MBT (BzMBT) to form, in the proposed reaction pathway, the intermediate peptide benzothiazole compound indicated at XIX. The formation of the intermediate may proceed via an activated anhydride with subsequent attack by a benzothiazole ion, or may involve a concerted rearrangement reaction, as discussed with reference to Figure 2.

The cyclization of the intermediate benzothiazole to form the peptidyl aryl TH likely proceeds along the same pathway as described in Figure 3, with the electrophilic thio ring carbon reacting with the amide nitrogen, followed by cyclization to form the desired peptide phenyl TH compound XIV.

Still another possible mixed anhydride is a mixed anhydride of isocyanic acid and carboxylic acid, such as benzoyl isocyanate. Such a reaction, which is described in Example 9, produces a reasonable yield of the desired amino acid-TH product, as reported in the example.

From the foregoing, it can be appreciated how various objects and features of the invention are achieved. The method of the invention permits peptidyl TH formation under substantially milder conditions than prior-art methods relying on anhydride activation of the peptide's carboxyl group. At the same time, the TH-formation reaction can be carried out relatively rapidly, e.g., 15-30 minutes, thus allowing sequencing to be carried out quickly in an automatic system.

In addition, when the cleavage reaction in the method is carried out under acidic conditions, the method preserves the stereochemistry of the C-terminal amino acid, and thus can be used to determine L- or D-form amino acids.

### II. Solid-Phase Method and Reagents

In a second general embodiment of the invention, formation of an amino acid TH is carried out by reacting an N-protected amino acid or peptide with a solid-phase mixed anhydride reagent. Specifically, a mixed anhydride of isothiocyanic acid and a carboxyl, carbonic or sulfonic acid and a solid support to which the mixed anhydride is derivatized. The mixed anhydride reagents used in this method are referred to herein as solid-phase reagents, and the general method in which an N-protected amino acid or peptide is reacted the solid-phase reagent, as a solid-phase method.

### IIA. Solid-Phase ITC Reagent

The method of the invention employs an acyl ITC solid-phase (immobilized) reagent which is derivatized with an acyl ITC, i.e., a mixed anhydride of a carboxylic or carbonic acid, and isothiocyanic acid. The reagent has the form: 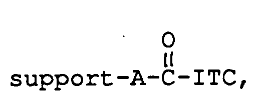
where A is an alkyl, alkoxy, aryl, or aryloxy group and ITC is isothiocyanate, also designated N=C=S or NCS in the figures. The alkyl group may be selected from the group of alkyl and cycloalkyl compounds, such as methyl, ethyl propyl, t-butyl, and related carbon-containing compounds linked to the acyl carbon through a carbon-carbon linkage. The aryl group may be a benzene, substituted benzene, or related aromatic or heteroaromatic compound linked to the acyl carbon through an aryl ring carbon atom. The alkoxy and aryloxy groups include analogous O-alkyl and O-aryl groups. The group of compounds indicated at A are also referred to herein as mixed anhydrides of ITC and carboxylic acids, since hydrolysis at the CO-N bond yields a carboxylic acid an ITC. The group of compounds indicated at B are likewise referred to herein as mixed anhydrides of isothiocyanic acid and carbonic acids, since hydrolytic cleavage of the compounds produce a carbonic acid ester and NCS.

The solid support may be particle beads, or a membrane support, or the like, derivatized with surface chemical groups by which the support can be functionalized to contain acyl ITC. A variety of bead or membrane support materials, such as glass or a number of polymer materials, such as nylon, polystyrene, polyethylene, Teflon™, having reactive chemical groups, such as amine, carboxyl, and hydroxyl groups which can be converted by a variety of known methods to the desired reactive ITCs are commercially available.

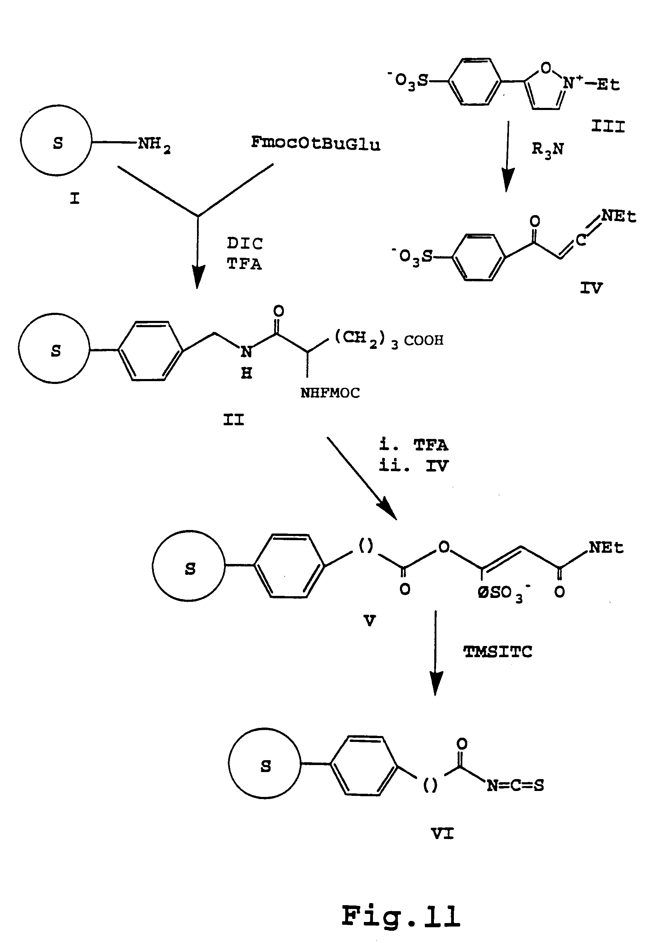

Figure 11 illustrates the steps in the formation of an activated solid support derivatized with acyl ITC groups. The solid support (I) is a particle bead or the like, indicated at S, having surface amine groups. The beads are first derivatized to contain carboxylic acid surface groups by reaction of the beads with a side chain-protected dicarboxylic acid such as FMOC-Glu-OtBu, and a carbodiimide reagent in a suitable solvent, to couple the dicarboxylic acid derivative to the amino surface groups through an amide linkage. The protected carboxylated support is shown at (II) in Figure 11. Reaction conditions are detailed in Example 10. The functional group is deprotected, e.g. with TFA.

It will be appreciated that the support could have been similarly prepared using other routes to generate a carboxylated support.

The support (II) is activated by reaction with an isoxazolium salt, such as Woodwards Reagent K (Woodward) (III). In the presence of a suitable base, such as a trialkyl amine, the isoxazolium salt forms a ketenimine (IV) which can react with the free carboxyl group of the support, to form an activated enol ester (V). The activated ester is reacted with an ITC compound, such as trimethylsilyl ITC (TMSITC), to form the desired activated acyl ITC solid support (VI). Specific reaction conditions are given in Example 10. Preferred ITC compounds trialkysilyl-ITC compounds, such as TMS-ITC, or pyridinium thiocyanate. The parentheses in structures V and VI represent the glutanic acid link in the support shown in structure II.

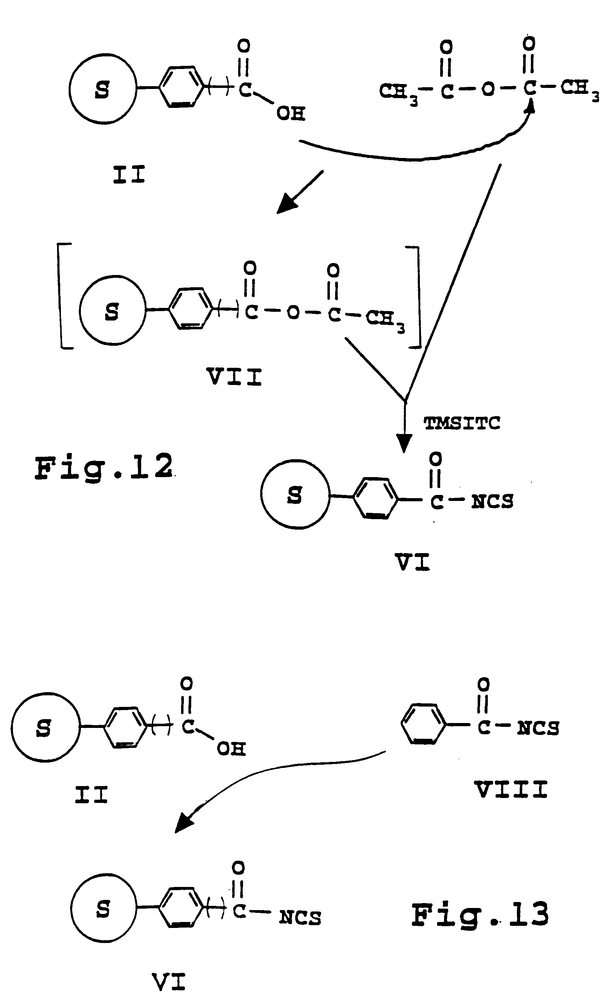

Figure 12 illustrates a second general method for forming a solid support activated with benzoyl ITC. Here support (II) is reacted with an anhydride, such as acetic anhydride, under anhydrous conditions, to form a presumed activated anhydride support (VII). Further reaction with an ITC compound, such as TMSITC, yields the desired benzoyl ITC activated support (VI).

A third method for forming an activated mixed anhydride ITC solid support is illustrated in Figure 13. In this method, a solution-phase acyl ITC compound, such as benzoyl ITC (VII), reacts directly with the carboxyl groups on the solid support to form the desired activated acyl ITC support. Details of the reaction are given in Example 12.

Solution-phase ITC compounds may be obtained commercially or prepared as described in the above-cited parent application. In one method of preparing acyl ITC, an ITC salt is added in a dry, inert solvent containing a base to an acid chloride.

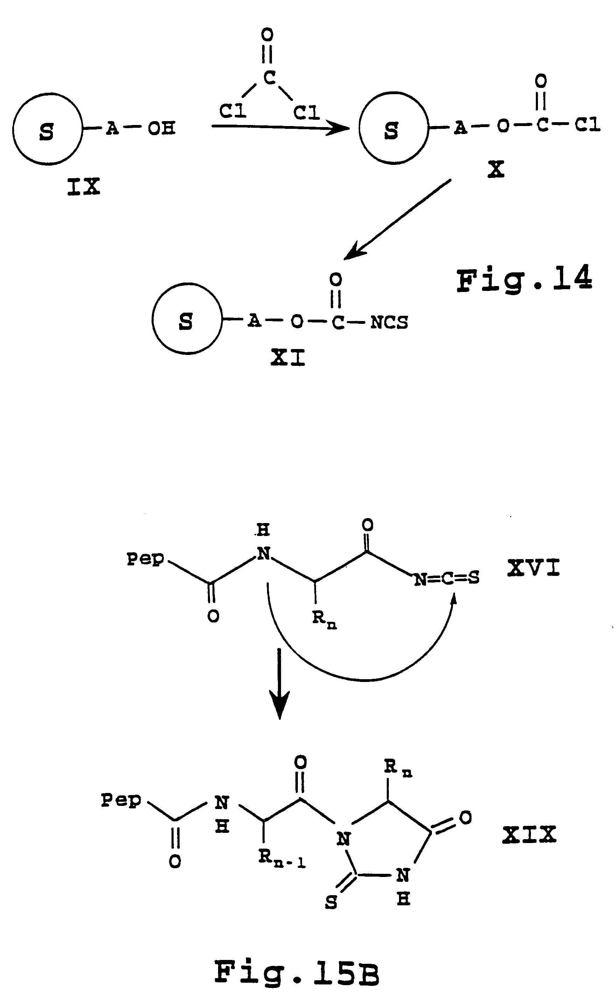

Figure 14 illustrates a method of forming an activated solid support derivatized with a mixed anhydride of a carbonic acid and ITC. The solid support (IX) used in the method is derivatized with alkyl or aryl (A) alcohol groups, according to known methods. This support is activated with a phosgene equivalent, such as triphosgene, to form the corresponding activated carbonate derivative (XI). Further reaction with an ITC, such as TMSITC, gives the desired activated support (XII).

The activated support may be stored in stable form dried or in an aprotic, non-nucleophilic solvent, such as benzene or toluene until used.

### IIB. Peptidyl-TH Formation

In practicing the method of the invention, a peptide whose C-terminal amino acid is to be identified is reacted with the acyl ITC reagent under basic conditions, preferably in the presence of pyridine, in which the C-terminal acid group of the amino acid is deprotonated. The C-terminal amino acid is converted to a C-terminal amino acid TH which is linked to the next-in C-terminal amino acid in the peptide through a ring-nitrogen amide bond.

The amino groups of a peptide are first protected with acetylation or formation of BOC (t-butyloxycarbonyl) or FMOC (fluorenylmethoxycarboxyl) derivatives, according to standard methods (Green). The N-protected peptide is dissolved in a suitable solvent, such 10% pyridine in acetonitrile, at a final peptide concentration typically between .1 and 10 »g/ml. The peptide solution is then added to the mixed-anhydride solid-phase reagent, preferably in molar excess of the activated mixed anhydride group. Typically, about per 100 »L of the peptide solution is added to 10 »g of the solid-phase reagent.

The TH-forming reaction is preferably carried out at 50-60°C, for about 10-60 minutes, preferably at about 50°C for 15-30 minutes, to minimize reaction of the ITC reagent with non-carboxyl groups in the peptide. Typical reaction conditions are given in Example 14.

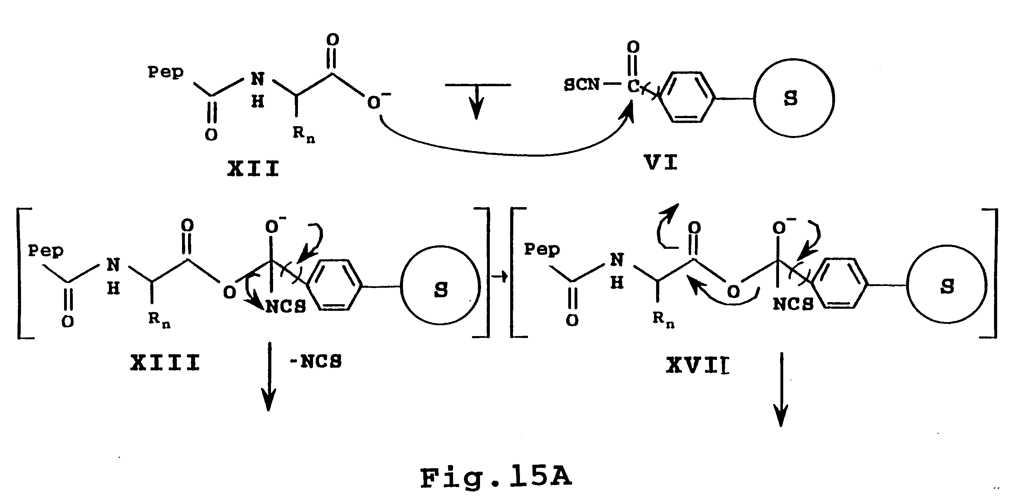

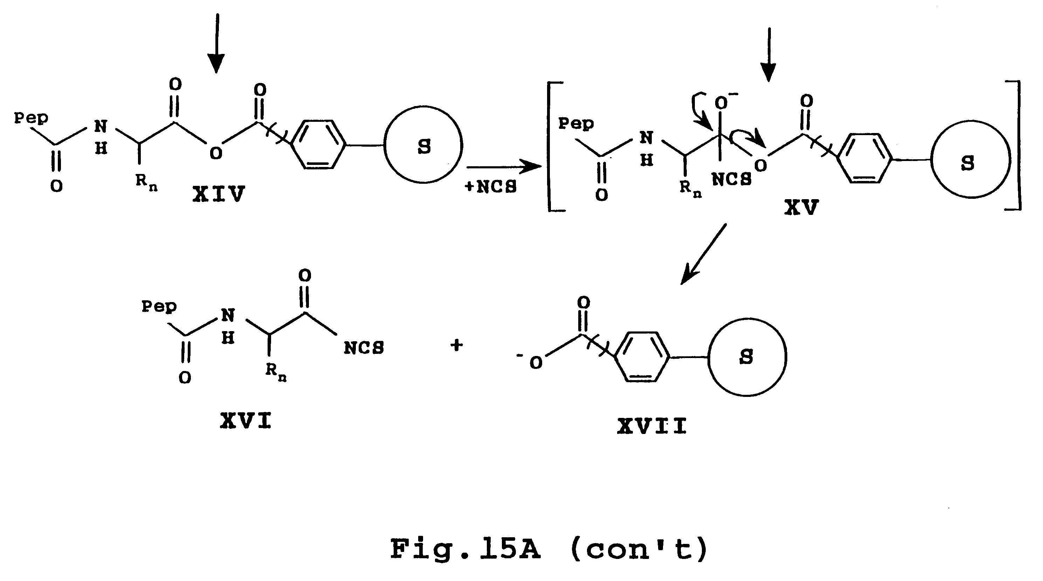

Figure 15 shows reaction mechanisms for the formation of a peptidyl TH by reaction with the activated support of the invention. In the mechanism shown at the left in Figure 15A, the peptide (XII) reacts at the carbonyl carbon of the mixed anhydride reagent (VI), through the intermediate XIII, to form the peptidyl acyl-ITC shown at XIV in the figure. The anhydride reacts with a free thiocyanate ion produced in the reaction, to form a peptidyl ITC (XVI). In this proposed mechanism, the mixed anhydride reagent functions both as an activating reagent, to form a reactive peptidyl anhydride, and as a source of thiocyanate ions for reaction with the anhydride.

An alternative reaction mechanism is shown at the right in Figure 15A. Here the thiocyanate group in the tetrahedral intermediate XVII in the figure migrates in a possibly converted fashion to the peptide carbonyl carbon, forming the tetrahedral intermediate XV which rapidly collapses to form the corresponding peptide ITC compound (XVI).

With continued reference to Figure 15A, there are two possible electrophilic sites of reaction of the peptidyl deprotonated (nucleophilic) oxygen atom. The first site, and the one shown in the Figure 15A reaction scheme, is the carbonyl carbon. The second site is the thiocarbonyl (thiocyanate) carbon. As reported in the earlier-filed parent application, reaction studies involving solution-phase mixed anhydrides indicate that the alkyl and aryl carbonyl groups (the mixed anhydrides of isothiocyanic acid and carboxylic acids) give the highest percentage of the desired carbon-site reaction product under the specified reaction conditions (in the presence of pyridine), and, correspondingly, the lowest percentage of reaction products resulting from nucleophilic attack at the thiocyanate carbon. The sulfonic acid mixed anhydride reagent gives the lowest percentage of the desired product.

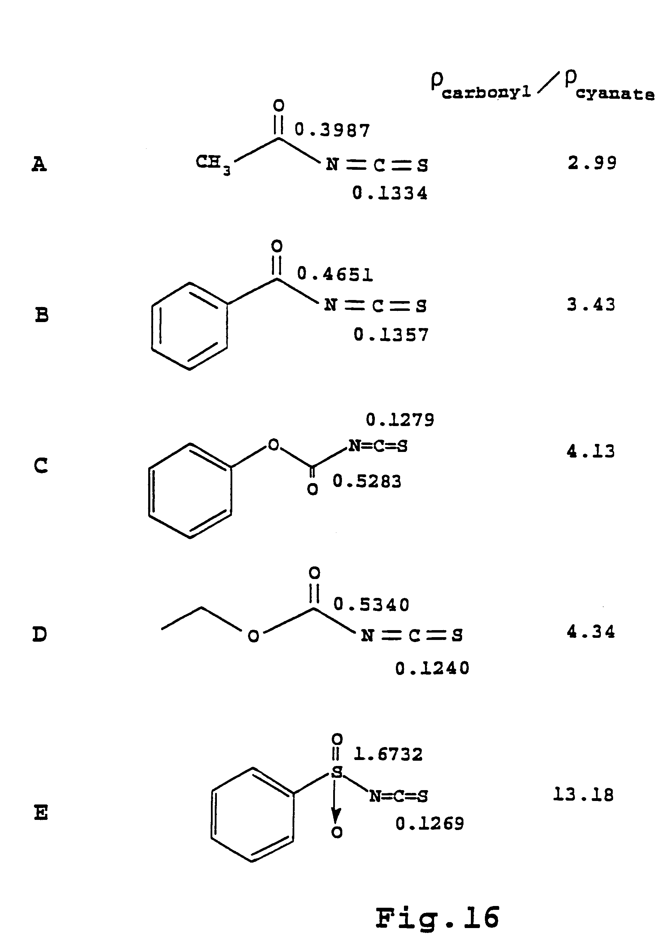

Molecular orbital calculations were carried out on the mixed anhydrides shown in Figure 16, which includes alkyl (A) and aryl (B) mixed anhydrides of carboxylic acid, alkyl (C) and aryl (D) anhydrides of carbonic acid, and an aryl mixed anhydride of sulfonic acid (E). The calculations were performed by the Modified Neglect of Diatomic Orbitals (MNDO) procedure (Dewar), using the MOPAC program available form the Quantum Chemistry Program Exchange (Bloomington, IN). The partial charges calculated for the carbonyl and cyanate carbon atoms by the program are shown for the five molecules in the figure, as well as the ratio of carbonyl/cyanate carbon-atom charge, arranged in ascending order. The calculations may be used to examine the likely effect of aryl or alkyl substitutions on the peptidyl TH reaction. For example, calculations of the charge ratios of benzoyl ITC's indicate that strongly electron withdrawing substituents, such as F or NO₂ on the phenyl ring have little effect on the relative partial charge ratio, indicating little effect on the ratio of the reaction products.

The yields of mixed products observed for the five compounds shown in Figure 16. The charge ratios calculated for the compounds, indicate that the cyanate carbon is activated by high partial charge at the carbonyl carbon (or sulfonyl sulfur), and thus becomes a more probable site for nucleophilic attack. This is analogous to the observed reactivity of an alpha-beta unsaturated ketone, where a greater partial charge at the ketone carbon leads to enhanced reactivity of the beta carbon toward nucleophilic attack. Although this reaction scheme might predict that the mixed anhydride of an alkyl carboxylic acid (e.g., compound A), would give best product yields, the higher reactivity of the alkyl carbonyl carbon may also make the mixed anhydride more susceptible to hydrolysis, and this may explain the somewhat greater yields of desired peptidyl-ITC product observed with the aryl carboxylic acid mixed anhydride.

The cyclization of the peptidyl ITC compound to form the desired peptidyl TH is shown in Figure 15B, and presumably involves a electrophilic attack by the thiocyanate carbon on the amide nitrogen in the peptide ITC compound, with rapid cyclization to form the peptidyl TH shown at XIX in the figure. The cyclization reaction may be catalyzed by pyridine, suggesting that pyridine may be reacting with the thiocyanate moiety to enhance the reactivity of the reactive carbon center, as has been proposed (Miller, 1989).

Following the reaction of the peptide with the activated solid support, the resultant peptidyl TH is separated from the solid support, by washing or eluting the reaction mixture from the solid support. According to an important feature of the invention, the only reaction components for converting a protected peptide to a peptidyl-TH are carried on the solid support, and no reaction products are released by the support in forming the peptidyl TH. That is, the peptidyl TH separated from the solid support is free of activating reagents or reagent byproducts of the TH-formation reaction.

Also as seen at the bottom of Figure 15A, the reaction regenerates the original (pre-activated) acid form of the solid support. The solid support can be reactivated by any of the activation reactions described above with respect to Figures 11-13.

In another aspect, the invention includes a method of producing an amino acid TH from an N-protected amino acid. The method is similar to that described above, substituting an N-protected amino acid for an N-protected peptide. Following formation of an amino acyl-ITC and cyclization to form the corresponding amino acid TH, the product is separated from the solid support, yielding a protected amino acid TH free of reactants and side reaction products. The isolated amino acid TH can be deprotected, e.g., under acid or base conditions, according to known methods.

### IIC. Formation and Identification of Amino Acid TH

This section describes the final steps of the method of the invention, which include (a) treating the peptidyl TH under cleavage conditions effective to cleave the amide linkage joining the TH to the residual peptide, and (b) isolating and identifying the isolated amino acid TH.

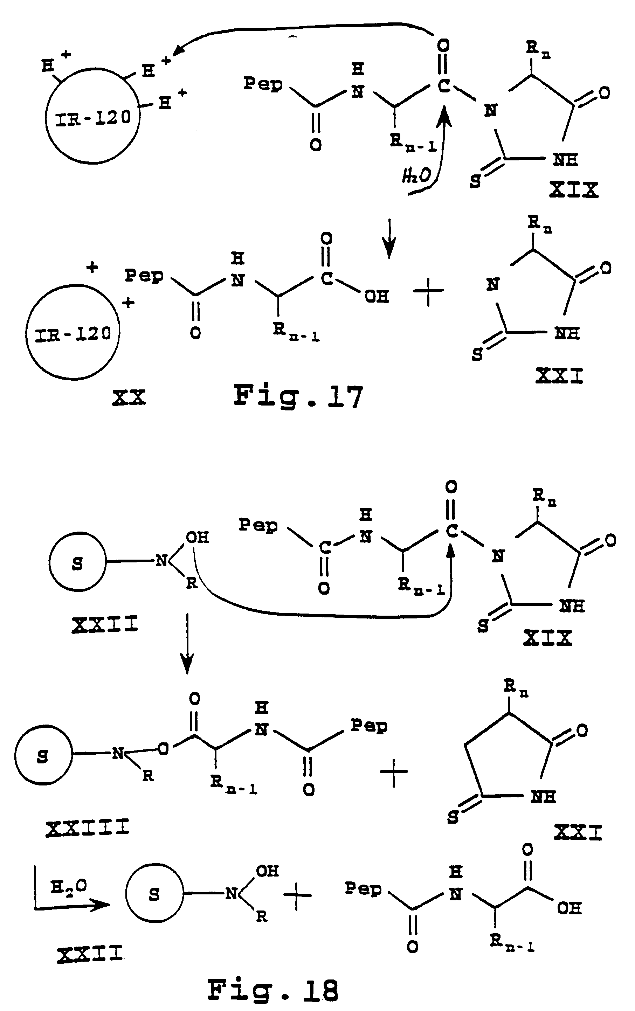

In a preferred method, the cleavage reaction is carried out by contacting the peptidyl TH in a suitable solvent with a solid-phase cleaving reagent. One solid-phase cleavage reagent which has been reported (Yamashita) is a cation-exchange resin Amberlite^{R} IR-120 (protonated form), available from Aldrich, (Milwaukee, WI). As illustrated in Figure 17, the resin promotes the acid-catalyzed hydrolysis of the peptidyl TH, yielding the desired amino acid TH (XXI) and residual peptide (XX). Typically, the peptidyl TH sample is added to the resin (about 2 mequiv H⁺ available per gram resin), and the mixture is incubated for 2-6 hours at room temperature. The peptide solution is prepared typically by removing the solvent used in peptidyl TH formation by vacuum, and redissolving the peptidyl TH in aqueous medium.

The residual peptide may be bound to the cation-exchange resin, allowing the free amino acid TH to be separated from the residual peptide, by washing the resin under low ionic strength conditions. With the residual peptide bound to the support, the free amino acid TH can be obtained by washing or elution in substantially purified form. The residual peptide may be released subsequently by eluting at elevated ionic strength or pH.

The residual peptide and amino acid TH formed as above may alternatively be separated, if necessary, by passage through a anion exchange resin, or by chromatography, for example, with by HPLC or molecular sieve chromatography.

In a second general embodiment, the cleavage reagent is derivatized with a chemical group capable of reacting with the peptidyl TH, to release free amino acid TH and link the residual peptide covalently. The free amino acid TH can then be isolated in substantially purified form by washing or elution, and the residual peptide can be released by hydrolytic cleavage of the peptide from the support.

One exemplary solid-phase cleavage reagent is the N-alkyl derivatized solid support (XXII) shown in Figure 18. The R group in the figure is preferably a methyl or other small alkyl group. As shown, hydrolytic cleavage of the peptidyl TH by the hydroxylamine group on the solid support forms an O-hydroxamate ester attachment of the residual peptide to the support, with the release of free amino acid TH. The reaction is preferably carried out in an aprotic solvent, such as MeCN, in the presence of an organic base, such as triethylamine (TEA).

After recovery of the amino acid TH, the residual peptide can be hydrolytically cleaved from the support by contact with acidified aqueous medium, as indicated at the bottom in Figure 18. Thus the method allows for isolation of amino acid TH and residual peptide separately, each in substantially purified form free of reactants and side products. The hydrolytic cleavage regenerates the hydroxylamine support.

The released amino acid TH compound may be identified by known chromatographic methods, such as high pressure liquid chromatography (HPLC), according to standard procedures. Compound identification can be made conveniently by comparing the run times in the columns with the run times of known reference amino acid TH's, prepared according to standard methods, or by the method of preparing an amino acid TH described above. Alternatively, the released and isolated amino acid TH can be identified by other available methods, such as mass spectrometry or NMR.

More generally, the invention contemplates hydrolytic cleavage of a peptidyl-TH reagent with a secondary hydroyl- amine having the general formula: 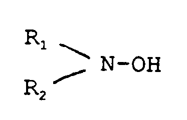
where R₁, R₂ are each alkyl or aryl groups, preferably alkyl, where one of the N substituents may be attached to a solid support. In this more general embodiment, a peptidyl-TH, either in solution or immobilized on a solid support, is reacted in water or in an organic solvent, such as acetonitrile, with the secondary hydroxyl amine which itself may be in solution (e.g., where the peptidyl-TH is immobilized), or immobilized (where the peptidyl-TH is in solution).

Preferred secondary hydroxylamines are dialkyhydroxylamines, such as dimethy- or diethyl-hydroxylamine, which are commercially available. Aryl hydroxyamines are also suitable, but may interfere with amino acid TH detection. Aryl hydroxylamines may also rearrange under acidic conditions to undesired amino phenols. R₁, R₂ may also be acyl groups, although these compounds may be overly susceptible to hydrolysis under aqueous conditions.

### IID. C-Terminal Solution-Phase Sequencing

This Section describes the application of the above method to a C-terminal solution-phase sequencing method which is suitable for automated or Semi-automated operation. As used herein, "solution phase sequencing" refers to sequencing reactions in which the peptide is retained in solution, and recycled successively through solid-phase reagents. This is in contrast to solid-phase sequencing, where the peptide is immobilized on a solid support, and is repeatedly exposed to solution-phase C-terminal residue activating and cleavage reagents.

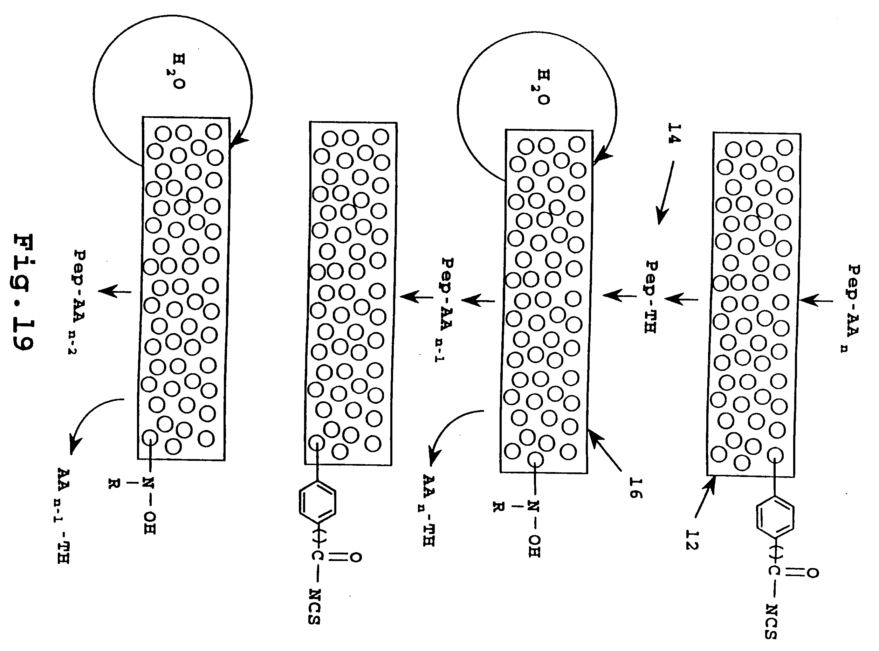

Figure 19 illustrates the general sequencing scheme as it is applied to a peptide Pep-AAₙ, i.e., a peptide having n residues and a C-terminal residue AAₙ. Initially, the peptide is dissolved in a suitable non-nucleophilic solvent such as pyridine in acetonitrile, and added to an activated solid-support resin contained in a packed column indicated at 12. Column 12 is preferably part of a two-stage cartridge 14 which also includes a column 16 containing a solid-support cleavage reagent.

After reacting the peptide with the activated support, the reaction solution, which contains peptidyl TH is eluted from column 12 into column 16. Optionally, the two columns may be separated by an intermediate chamber (not shown), where the column 12 solvent may be removed by vacuum and replaced by a second solvent before sample introduction into column 16. In a preferred embodiment, the solid-support reagent is the type illustrated in Figure 18. This support has the advantages that (a) the cleavage reaction can be carried out in the same non-aqueous solvent or solvent mixture used in the column-12 reaction, and (b) the binding to and release from the support of the residual peptide to the support is covalent, and therefore not dependent on the charge characteristics of the peptide.

The cleavage reaction is carried out under conditions as described above, after which the C-terminal amino acid TH (AAₙ-TH) is eluted from the column and identified, e.g., by HPLC. Subsequently, the column is washed with an aqueous medium to release the bound residual peptide (Pep-AAₙ₋₁).

If the reaction columns are to be reused, they are washed extensively, and the first column is reactivated to an acyl ITC form, as described above. Alternatively, the two-column cartridges may be disposable, with additional sequencing reactions occurring in fresh cartridges.

The residual peptide obtained from the first sequencing cycle is dried, redissolved in a suitable reaction buffer, and introduced into a new or reactivated cartridge. The second sequencing cycle yields an amino acid TH of the penultimate C-terminal residue (AAₙ₋₁-TH) and a residual peptide shortened by two C-terminal residues. The sequencing cycle is repeated until the desired number of C-terminal residues have been identified. The total number of C-terminal residues which can be identified with high confidence will vary according to the degree to which the peptide is converted to the desired peptidyl-TH in the first reaction column, and the degree to which the peptidyl-TH is cleaved in the second reaction column. In general, the reaction conditions will be selected to enhance product yields where more 3-5 residue sequencing is desired.

In another aspect, the invention includes a solid-phase system for use in determining the C-terminal amino acid residue of a peptide. The system includes an activated support composed of a solid support derivatized with a mixed anhydride of isothiocyanic acid and carboxylic or carbonic acid, and a second support composed of a solid support derivatized with a cleaving agent which is effective to a cleave a peptidyl hydantoin to form a free amino acid thiohydantoin and a residual peptide. Preferred supports are particle supports, e.g., packed in a column, or filter membranes through which the peptide solution may be passed.

From the foregoing, it can be appreciated how various objects and features of this embodiment of the invention are achieved. The method of peptidyl TH formation provides the advantages of the acyl ITC reaction method disclosed in the above-cited parent application. Specifically: the reaction (a) is relatively rapid, (b) can be carried out under mild reaction conditions, and (c) where the cleavage reaction is carried out under acidic conditions, preserves the stereochemistry of the C-terminal amino acid, and thus can be used to determine L- or D-form amino acids.

The following examples are intended to illustrate the synthesis of various acyl compounds, and their use in determining C-terminal amino acid groups, and for C-terminal sequencing. The examples are in no way intended to limit the scope of the invention.

### Materials

Pyridine, methylene chloride, ethylene oxide, BF₃, ethyl ether, triethylamine, Woodwards Reagent K, trimethylsilyl ITC, triphosgene, and benzoyl ITC were obtained from Aldrich (Milwaukee, WI). Leucine enkephalin was from Sigma, dipeptides were from Bachem Biosciences. Polydimethyl acrylamide resin was prepared according to J. Sparrow, and had about 0.7 meq/g of amino groups. Conversion of those amino groups to isothiocyanates was performed using thiocarbonyl diimidazole and diisopropylethyl amine (DIPEA) in acetonitrile. Nuclear magnetic resonance spectra were collected on a Jeol? HX90 spectrometer. Mass spectral analyses were performed by the Berkeley Mass Spectrometry Laboratory under the direction of Dr. J. Leary.

Peptides were attached to resin in one of two ways: (1) by a urea linkage, or (2) by a thiourea linkage (using isothiocyanato-resin, above).
(1). Urea link: The resin is neutralized with excess DIPEA and allowed to react with excess DSC in 10% pyridine/NMP for 1-2 hours at ambient temperature. After several washes, finally with ACN, a solution of the peptide in 10% pyridine/water is added to the moist resin and allowed to stand overnight. The resin is washed extensively and dried under vacuum. Typical loading is >100 nanomoles/ml. Confirmation is by amino acid analysis, and one round of C-terminal analysis.
(2). Thiourea link: A solution of the peptide is added to either ITC (above) or DITC resin, and incubated at 45° C overnight. After rinsing and drying, attachment is confirmed by TFA cleavage of the peptide (less the aminoterminal amino acid) from the resin followed by characterization on HPLC and by sequence analysis.

### Example 1

### C-Terminal Analysis of a Leu-Val Dipeptide

### A. Coupling:

Peptidyl resin (about 1 mg, Leu-Val attached via a urea-linkage) was suspended in 100 »l of 10% pyridine/acetonitrile. Benzoyl isothiocyanate (BITC) (10 »l) was added, mixed by agitation, and allowed to react for 30 minutes at 60°C. The resin was extensively washed with several volumes of acetonitrile, and dried under vacuum.

### B. Hydrolysis:

The resin from above was wetted with 10 »l of 10% propylamine in acetonitrile. After 15 minutes at room temperature volatiles were removed and the amino acid TH extracted with acetonitrile for analysis.

### C. HPLC:

The separation of the hydrophobic amino acid thiohydantoins was readily achieved on a narrow-bore system (Model 120A, Applied Biosystems) using a PTH-C18 column (2.1 mm x 22 cm, ABI) and a TFA-water-acetonitrile gradient system. The column was first equilibrated in A solvent (0.1% TFA in water, v/v), held in 100$ A for 5 minutes after injection, then a gradient was developed to x% B solvent (0.85% TEA in 70% acetonitrile) over x minutes. The percentage of B was then increased to 90% over 5 minutes, and held there for 20 minutes. he flow rate was 200 »l/min at ambient temperature. Effluent was monitored at 269 nm for TH, and at 214 nm for peptides.

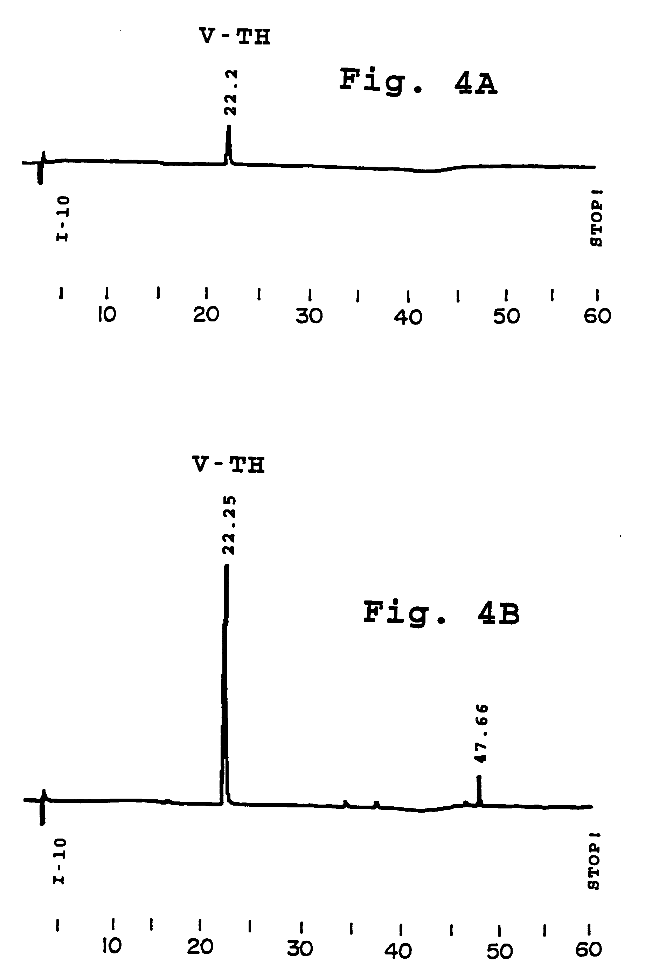

The thiohydantoin was identified by comparison with the elution of authentic material (see part D, below). The HPLC of the extract in part B is shown in figure 4B. The major peak is readily identified as TH-V. The assignment was confirmed by coinjection. Figure 4A shows the TH-V product generated in a control experiment in which the resin was reacted with trimethylsilyl isothiocyanate (Hawke).

### D. Preparation of Authentic Standards:

Amino acid thiohydantoins of the hydrophobic residues are preparable by classical methods (such as Cromwell). In brief, the amino acid is treated with a thiocyanate salt in acetic acid/acetic anhydride at up to 90° C for 30 minutes. After vacuum drying, the residue was taken up in 12 N HCl and allowed to stand at room temperature for up to 1 hour. The mixture was again vacuum dried, and the residue recrystallized, usually from boiling water. The structures were consistent by NMR and mass spectrometry.

### Example 2

### C-Terminal Analysis of Ala-Met Dipeptide

Following the procedures described in Example 1, immobilized Ala-Met (urea linkage) was degraded for one cycle using BITC and PA. The resulting chromatogram is shown in Figure 4D. Methionine TH (M-TH) assignment was confirmed by coinjection.

### Example 3

### Leucine Enkephalin C-Terminal Analysis

Leu-enkephalin (YGGFL) linked through a thiourea linkage to resin was treated with BITC and PA as in Example 1. The released L-TH was detected by HPLC.

The resin was further rinsed with acetonitrile and dried, then treated with 2% TFA in water at 60°C for 10 minutes to release the peptide components from the resin. HPLC analysis followed by isolation and both sequencing and FAB mass spectrometry showed a major peak corresponding to the desleucine peptide, confirming the loss of the C-terminal amino acid.

### Example 4

### C-Terminal Analysis of N-Protected Phe Gly

N-protected t-Boc Phe-Gly was obtained commercially. The peptide was suspended in 100 »l of 10% pyridine/acetonitrile (PA). Trimethylacetyl ITC (10 »l) was added, mixed by agitation, and allowed to react for 30 minutes at 60°C. The resin was extensively washed with several volumes of acetonitrile, and dried under vacuum.

Cleavage of both the t-Boc group and the Gly-TH was achieved by heating at 60° in 25% TFA/water. The reaction, as followed by HPLC, showed rapid loss of starting material (judged after 15 minutes of reaction), along with the appearance of G-TH and a minor component (probably the Boc-deprotected depeptidyl-TH). After two hours reaction, only G-TH was detectable.

### Example 5

### C-Terminal Analysis of Leu-Enkephalin

Immobilized leucine enkephalin (urea linkage) was degraded for two cycles of chemistry. The couplings were performed with ethoxycarbonyl isothiocyanate (instead of BITC) for 5 minutes at 60° C. Cleavages were effected by adding 10 »l of 10% tetra-N-butyl ammonium hydroxide in water containing 1 mg/ml DTT to the dry resin, and heating for 45 minutes at 60° C. Chromatograms are shown in Figs. 5A and 5B. The cycles were assigned as leucine and phenylalanine, respectively. The assignments were confirmed by coinjection.

### Example 6

### Preparation of Single Isomer of Isoleucine

Approximately 10 »moles of t-Boc-isoleucine was dissolved in 100 »l 10% pyridine/acetonitrile. This was heated with 10 »l BITC at 60° for 20 minutes. HPLC analysis of the product showed a single major new peak, the t-Boc-Ile-thiohydantoin. A small portion of this mixture was cleaved in 25% TFA at 60° C for 10 minutes. The reaction mixture was diluted with acetonitrile and analyzed by HPLC. The result was a single peak corresponding to Ile-TH. Running the peak isocratically (11% B) gave an improved resolution of isomers, shown in Fig. 7B, and allows estimation of the upper bound of about 2% epimerization.

T-Boc-isoleucine (10 »mol) was dissolved in 100 »l 10% TMS-ITC in acetic anhydride. The reaction was heated at 60° for 20 minutes. The material was cleaved in 25% TFA at 60° C for 10 minutes, and the reaction mixture was diluted with acetonitrile and analyzed by HPLC, as above. The result was a doublet peak, corresponding to the diasteromeric forms of Ile-TH, as seen in Fig. 7A.

### Example 7

### Reaction of Sulfonyl ITC Reagent with N-Protected Amino Acid

### A. Preparation of benzenesulfonyl isothiocyanate (BzS-ITC)

Benzenesulfonyl chloride was obtained from Aldrich Chemical Co. The compound (1mmol) was dissolved in 10 ml CH₂Cl₂ with 3 mmol pyridine ethylamine to a final volume. to this mixture was added 1 mmol of TMSITC, and the reaction mixture wa stirred for 1 hour at 25° C. The benzylsulfonyl isothiocyanate (BzSITC) was recovered by filtering the salts which formed, and removing the solvent and volatile side-products by vacuum.

### B. Reaction to form amino acid TH

One mmol of t-Boc-Leu was dissolved in dichloromethane containing pyridine, and 1 mmol of BzSITC prepared as in A. above. After reacting overnight, the solvent was removed by rotary evaporation and the t-Boc-Leu-TH which formed was purified by chromatography on silica gel. The identity of the TH compound was confirmed by NMR and HPLC.

### Example 8

### Reaction of BzMBT with Peptidyl Resin

### A. Preparation of BzMBT

Mercaptobenzothiazole (1 mmole) and diisopropyl ethylamine (1 mmole) were dissolved in 5 ml acetonitrile. Benzoyl chloride (1 mmole) was added slowly by syringe while stirring rapidly. After 2 hours at room temperature solvent was partially removed to permit precipitation of the amine salt. The supernatant was further concentrated, and chromatographed on silica gel (9:1, heptane:ethyl acetate).

### B. Reaction

Peptidyl resin (1 mg Lenk, thiourea linkage) was suspended in 100 »l of 10% pyridine in acetonitrile containing 1 mg Bz-MBT. The reaction was heated at 60° C. for 30 minutes, then washed and dried as before. The arylthiohydantoin was cleaved with 10% propylamine at room temperature for 15 minutes. Treatment with TFA released the remaining peptide fragments from the resin, as confirmed by the loss of the C-terminal residue as assessed by HPLC.

### Example 9

### Reaction with Benzoyl Isothiocyanate

One mole of t-Boc-Leu was reacted with a slight excess of benzoyl isothiocyanate in a reaction mixture similar to that in Example 7. The t-Boc-Leu was chromatographed, then deprotected to give a moderate yield of hydantoin, as confirmed by NMR.

### Example 10

### Preparation of Activated Solid Support: Method 1

The p-aminomethylpolystyrene resin (1% cross-linked divinylbenzene) (2 g, 1.10 mmole/g) was preswelled in CH₂Cl₂. FMOC-Glu-OtBu (3 mmole, 0.8 mole excess) was dissolved in 10 mL CH₂Cl₂ and added to the resin. N, N-diisopropylcarbodiimide (0.46 mL, 3 mole) was added immediately and the reaction was shaken for approximately 1 hour. The resin was washed well with solvent and an aliquot tested with ninhydrin (no amine was left). The tBu protecting group on the gamma carboxyl was removed with trifluoroacetic acid TFA (50% H₂Cl₂/TFA) in approximately 0.4 hr. The resin was washed with solvent and neutralized with diisopropylethylamine (DIPEA).

Meanwhile, Woodwards Reagent K (WRK, 3 Mmole) was converted to the ketenimine with 3 equivalents in 20 mL CH₂Cl₂ over approximately 2 hours. The solution became nearly homogenous and yellow, and was then added to the polystyrene resin. After two hours, the resin was washed and 3 mmole of TMSITC was added. After stirring overnight, the resin was washed with CH₂Cl₂. The functionalized resin gained 0.74g in weight. Theoretical weight gain would have been 0.87 g.

### Example 11

### Preparation of Ala-TH

BOC-Ala (10 mg) was dissolved in 2 mL CH₂Cl₂ and 12 »L of pyridine. This was incubated at 40°C over 100 mg of freshly prepared resin. The solution phase was pipetted off of the resin and concentrated by vacuum centrifugation. The residual oil was checked by HPLC and ¹H NMR. The integration indicated approximately 50% of the BOC-Ala had been converted to the corresponding TH. The BOC group was removed with aqueous TFA. The HPLC retention time agreed with that of authentic AlaTH.

The amino acid TH formed was identified by HPLC, using a narrow-bore system (Model 120A, Applied Biosystems) using a PTH-C18 column (2.1 mm x 22 cm, ABI) and a TFA-water-acetonitrile gradient system. The column was first equilibrated in A solvent (0.1% TFA in water, v/v), held in 100% A, 0% solvent B for 5 minutes after injection, then a linear gradient was developed to 40% B solvent (0.85% TFA in 70% acetonitrile) over 30 minutes. The percentage of B was then increased to 90% over 5 minutes, and held there for 20 minutes. The flow rate was 200 »l/min at ambient temperature. Effluent was monitored at 269 nm.

## Claims

1. A method of producing a thiohydantoin (TH) of a protected free or C-terminal amino acid having a free carboxylic acid group comprising
contacting the amino acid in solution with a mixed anhydride of isothiocyanic acid and carboxylic, carbonic, or sulfonic acid, under basic conditions in which the carboxylic acid group of the amino acid is deprotonated, and
by said contacting, forming the TH of the amino acid.

2. The method of claim 1, wherein the mixed anhydride is in solution, and has the form: 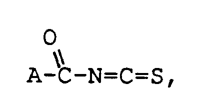 A is an alkyl, alkoxy, aryl, or aryloxy group.

3. The method of claim 2, wherein said mixed anhydride is a benzoyl isothiocyanate.

4. The method of claim 2, for use in determining the C-terminal amino acid of a peptide, wherein said contacting is effective to produce a C-terminal peptidyl thiohydantoin in which a C-terminal amino acid thiodantoin is linked to the penultimate C-terminal amino acid in the peptide through an amide bond, and which further includes treating the peptidyl thiohydantoin under conditions effective to cleave said amide bond, thereby releasing a C-terminal amino acid thiohydantoin and a reduced-length peptide, and identifying the released amino acid thiohydantoin.

5. The method of claim 4, wherein said peptide is attached at a non C-terminal residue to a solid support, and which further includes repeating said contacting, forming, treating and identifying steps one or more times to identify the sequence of amino acids in the peptide, from the C-terminal peptide end.

6. The method of claim 1, wherein the mixed anhydride is derivatized to a solid support, and has the form: 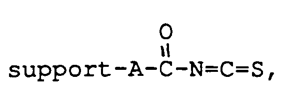 A is an alkyl, alkoxy, aryl, or aryloxy group attached to the solid support, and the protected free or C-terminal amino acid is in solution.

7. The method of claim 6, which further includes separating the amino acid TH formed by said contacting from the solid support.

8. The method of claim 7, wherein the amino acid is the C-terminal amino acid in a peptide, said contacting produces a solution-phase peptidyl TH in which the C-terminal peptide is an amino acid TH and said separating includes separating the peptidyl TH from said solid support, cleaving the C-terminal amino acid TH from the residual peptide, and isolating the free amino acid TH free of the residual peptide.

9. The method of claim 8, for use in C-terminal peptide sequencing, which further includes identifying the amino acid TH to determine the C-terminal amino acid of the peptide.

10. The method of claim 8, wherein said cleaving includes contacting a solution of the peptidyl TH with a second solid support derivatized with a cleaving agent which is effective to cleave the peptidyl TH to form the free amino acid TH and the residual peptide, both in solution phase.

11. An activated support comprising a solid support derivatized with a mixed anhydride of isothiocyanic acid and carboxylic or carbonic acid.

12. The support of claim 11, which has the form: 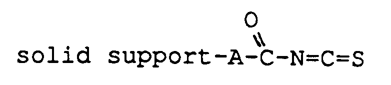 where A is an alkyl, alkoxyl, aryl or aryloxy group attached to the solid support.

13. A solid-phase system for use in determining the C-terminal amino acid residue of a peptide, comprising
an activated support composed of a solid support derivatized with a mixed anhydride of isothiocyanic acid and carboxylic or carbonic acid, and
a second support composed of a solid support derivatized with a cleaving agent which is effective to a cleave a peptidyl TH to form a free amino acid TH and a residual peptide.

14. The system of claim 13, wherein the second support is an ion exchange resin effective to selectively bind the residual peptide under selected ionic strength and pH conditions.

15. The system of claim 14, wherein the second solid support is derivatized with a hydroxylamine group effective to (a) cleave the peptidyl TH to form the free amino acid TH and (b) covalently bound to the residual peptide.

16. A method of preparing an acyl isothiocyanate effective to convert a peptide, when contacted with the support, into a C-terminal peptidyl thiohydantoin (TH), comprising
providing a solid support functionalized with carboxyl groups,
reacting the solid support with an isoxazolium compound under basic conditions, to form activated enol esters of the carboxyl groups, and
reacting the support with an isothiocyanate (ITC) selected from the group consisting of trialkylsilyl ITC and pyridine-ITC, to form a mixed anhydride of isothiocyanic acid and carboxylic acid on the support.

17. The method of claim 16, wherein said ITC is trimethyl-ITC.

18. The method of claim 16, wherein the isoxazolium compound is 2-ethyl-5'phenylisoxazoliumsulfonate.

## Patentansprüche

1. Ein Verfahren zur Herstellung einer Thiohydantoin (TH) einer geschützten freien Aminosäure oder einer C-Ende-Aminosäure mit einer freien Carboxylsäuregruppe, aufweisend
das Kontaktieren der Aminosäure in Lösung mit einem gemischten Anhydrid der Isothiocynansäure und Carbonsäure, Kohlensäure oder Sulfonsäure, und zwar unter Basisbedingungen, in welchen die Karbolsäuregruppe der Aminosäure de-protoniert ist und
Bilden der TH der Aminosäure durch das Kontaktieren.

2. Das Verfahren nach Anspruch 1, wobei das gemischte Anhydrid in Lösung ist und die Form hat: 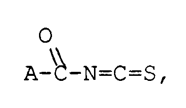 wobei A eine Alkyl-, eine Alkoxy-, eine Aryl- oder eine Aryloxygruppe darstellt.

3. Das Verfahren nach Anspruch 2, wobei das gemischte Anydrid ein Benzoylisothiocyanat ist.

4. Das Verfahren nach Anspruch 2 zur Verwendung der Bestimmung der C-Ende-Aminosäure eines Peptids, wobei das Kontaktieren wirksam ist, um ein C-Ende-Peptidylthiohydatoin zu bilden, in welchem eine C-Ende-Aminosäurethiodantoin mit der zum C-Ende vorletzten Aminosäure in dem Peptid über eine Amidbindung gebunden ist, und weiterhin umfassend die Behandlung des Peptidylthiohydantoin unter Bedingungen, die wirksam sind die Amidbindung aufzutrennen, um dadurch eine C-Ende-Aminosäurethiohydantoin und ein Peptid verringerter Länge freizugeben und das Identifizieren der freigegebenen Aminosäurethiohydantoin.

5. Das Verfahren nach Anspruch 4, wobei das Peptid an einem Nicht-C-Ende-Rest mit einem festen Träger verbunden ist und weiter das ein oder mehrmalige Wiederholen der Kontaktier, der Bildungs-, der Behandlungs- und der Identifizierungsstufe umfaßt, um die Sequenz der Aminosäuren in dem Peptid von dem C-Ende-Peptidendstück her zu identifizieren.

6. Das Verfahren nach Anspruch 1, wobei das gemischte Anhydrid mit einem festen Träger derivatisiert ist und die Form hat: 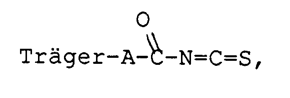 wobei A eine Alkyl-, eine Alkoxy-, eine Aryl- oder eine Aryloxygruppe darstellt, die an dem festen Träger befestigt ist, und wobei die geschützte freie Aminosäure oder die C-Ende-Aminosäure sich in Lösung befindet.

7. Das Verfahren nach Anspruch 6, weiterhin gekennzeichnet durch die Separation der Aminosäure-TH, die durch das Kontaktieren mit dem festen Träger gebildet ist.

8. Das Verfahren nach Anspruch 7, wobei die Aminosöäure die C-Ende-Aminosäure in einem Peptid ist, wobei das Kontaktieren eine Lösungshphase-Peptyl-TH bildet, in welcher das C-End-Peptid eine Aminosäure TH ist und die Separation die Trennung der Peptidyl-Th von dem festen Träger, das Abspalten der C-Ende-Aminosäure-TH von dem Restpeptid und die Isolierung der freien Aminosäure TH von dem Restpeptid umfaßt.

9. Das Verfahren nach Anspruch 8 zum Einsatz bei der C-Ende-Peptidsequenzierung, weiterhin die Stufe der Identifizierung der Aminosäure-TH umfassend, um die C-Ende Aminosäure des Peptids zu bestimmen.

10. Das Verfahren nach Anspruch 8, wobei das Abspalten das Kontaktieren einer Lösung der Peptidyl-TH mit einem zweiten festen Träger umfaßt, der mit einem Trennagenz derivatisiert ist, welches wirksam ist, um die Peptidyl-TH abzutrennen, um die freie Aminosäure-TH und das Restpeptid, beide in der Lösungsphase, zu bilden.

11. Ein aktivierter Träger, der einen festen Träger umfaßt, der mit einem gemischten Anhydrid der Isothiocyansäure und Carbon- oder Kohlensäure derivatisiert ist.

12. Der Träger nach Anspruch 11, welcher die Form hat; 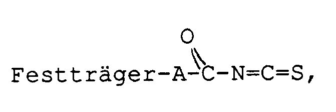 wobei A eine Alkyl-, eine Alkoxy-, eine Aryl- oder eine Aryloxygruppe darstellt.

13. Ein Festphasensystem zur Verwendung bei der Bestimmung des C-Ende-Aminosäurerestes eines Peptids, aufweisend
einen aktivierten Träger, der aus einem festen Träger gebildet ist, der mit einem gemischten Anhydrid der Isothiocyansäure und Carbon- oder Kohlensäuresäure derivatisiert ist, und
einen zweiten Träger, der aus einem festen Träger gebildet ist, der mit einem Trennagenz derivatisiert ist, welches wirksam ist, um eine Peptidyl-TH abzutrennen, um eine freie Aminosäure TH und ein Restpeptid zu bilden.

14. Das System nach Anspruch 15, wobei der zweite Träger ein Ionenaustauschharz ist, welches wirksam ist, um selektiv das Restpeptid unter geeignet ausgewählter Ionenstärke und pH-Bedingungen zu binden.

15. Das System nach Anspruch 14, wobei der zweite feste Träger mit einer Hydroxylamingruppe derivatisiert ist, welche wirksam ist, um (a) die Peptidyl -TH abzuspalten, um eine freie Aminosäure- TH zu bilden, und um (b) diese kovalent an dem Restpeptid zu binden.

16. Verfahren zur Präparation eines Acylisothiocyanats, welches wirksam ist, um ein Peptid, wenn es mit dem Träger in Berührung tritt, in ein C-Ende-Peptidylthiohydantoin (TH) umzuwandeln, aufweisend
Bereitstellen eines festen Trägers, der mit Carboxlygruppen funktionalisiert ist,
Reagieren des festen Trägers mit einer Isoxazoliumzusammensetzung unter Basisbedingungen, um aktivierte Enolester der Carboxylgruppen zu bilden, und
Reagieren des Trägers mit einem Isothiocyanat (ITC), welche aus der Gruppe ausgewählt ist, welche Trialkylsilyl-ITC und Pyridin-ITC umfaßt, um ein gemischtes Anhydrid der Isothiacyansäure und der Carbonsäure auf dem Träger zu bilden.

17. Das Verfahren nach Anspruch 16, wobei das ITC Trimethyl-ITC ist.

18. Das Vefahren nach Anspruch 16, wobei das Isoxazolium 2-ethyl-5'-phenylisoxazoliumsulfonat ist.

## Revendications

1. Procédé pour produire une thiohydantoïne (TH) d'un acide aminé libre ou C-terminal, protégé, ayant un groupe acide carboxylique libre, qui comprend
la mise en contact l'acide aminé en solution avec un anhydride mixte d'acide isothiocyanique et d'un acide carboxylique, carbonique ou sulfonique, dans des conditions basiques dans lesquelles le groupe acide carboxylique de l'acide aminé est déprotoné, et
grâce à cette mise en contact, la formation de la TH de l'acide aminé.

2. Procédé selon la revendication 1, dans lequel l'anhydride mixte est en solution et a la forme : 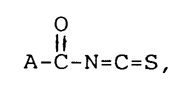 où A est un groupe alkyle, alcoxy, aryle ou aryloxy.

3. Procédé selon la revendication 2, dans lequel l'anhydride mixte est un isothiocyanate de benzoyle.

4. Procédé selon la revendication 2, pour déterminer l'acide aminé C-terminal d'un peptide, dans lequel ladite mise en contact permet de produire une peptidyl thiohydantoïne C-terminale dans laquelle une thiohydantoïne de l'acide aminé C-terminal est liée à l'acide aminé C-préterminal se trouvant dans le peptide, par l'intermédiaire d'une liaison amide, et qui comprend en outre le traitement de la peptidyl thiohydantoïne dans des conditions conduisant au clivage de ladite liaison amide, de façon à libérer une thiohydantoïne d'acide aminé C-terminal et un peptide de longueur réduite, et l'identification de la thiohydantoïne d'acide aminé ainsi libérée.

5. Procédé selon la revendication 4, dans lequel ledit peptide est fixé, au niveau d'un résidu non-C-terminal, à un support solide, et qui comprend en outre la répétition des étapes de mise en contact, de formation, de traitement et d'identification, à une ou plusieurs reprises, pour identifier, à partir de l'extrémité C-terminal du peptide, la séquence d'acides aminés du peptide.

6. Procédé selon la revendication 1, dans lequel l'anhydride mixte est fixé à un support solide et a la forme 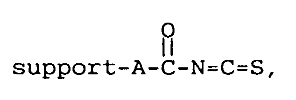 où A est un groupe alkyle, alcoxy, aryle ou aryloxy fixé au support solide, et l'acide aminé libre ou C-terminal, protégé, est en solution.

7. Procédé selon la revendication 6, qui comprend en outre la séparation de la TH d'acide aminé formée par ladite mise en contact, à partir du support solide.

8. Procédé selon la revendication 7, dans lequel l'acide aminé est l'acide aminé C-terminal d'un peptide, ladite mise en contact produit une peptidyl TH en solution, où le peptide C-terminal est une TH d'acide aminé, et ladite séparation comprend la séparation de la peptidyl TH dudit support solide, le clivage de la TH d'acide aminé C-terminal à partir du peptide résiduel et l'isolement de la TH d'acide aminé libre du peptide résiduel.

9. Procédé selon la revendication 8, pour utilisation pour le séquençage d'un peptide C-terminal, qui comprend en outre l'identification de la TH d'acide aminé pour déterminer l'acide aminé C-terminal du peptide.

10. Procédé selon la revendication 8, dans lequel ledit clivage comprend la mise en contact d'une solution de la peptidyl TH avec un deuxième support solide transformé par un agent de clivage qui permet de cliver la peptidyl TH pour former la TH d'acide aminé libre et le peptide résiduel, les deux étant en solution.

11. Support activé comprenant un support solide transformé par un anhydride mixte d'acide isothiocyanique et d'un acide carboxylique ou carbonique.

12. Support selon la revendication 11, qui a la forme : 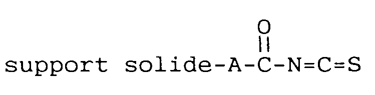 où A est un groupe alkyle, alcoxy, aryle ou aryloxy fixé au support solide.

13. Système en phase solide pour utilisation pour la détermination du résidu d'acide aminé C-terminal d'un peptide, qui comprend :
un support activé constitué d'un support solide transformé par un anhydride mixte d'acide isothiocyanique et d'un acide carboxylique ou carbonique, et
un deuxième support constitué d'un support solide transformé par un agent de clivage qui permet de cliver une peptidyl TH pour former une TH d'acide aminé libre et un peptide résiduel.

14. Système selon la revendication 13, dans lequel le deuxième support est une résine échangeuse d'ions pouvant fixer sélectivement le peptide résiduel dans des conditions sélectionnées de force ionique et de pH.

15. Système selon la revendication 14, dans lequel le deuxième support solide est transformé par un groupe hydroxylamine pouvant (a) cliver la peptidyl TH pour former la TH d'acide aminé libre, et (b) se lier par liaison covalente au peptide résiduel.

16. Procédé pour préparer un isothiocyanate d'acyle efficace pour convertir un peptide, quand il est mis en contact avec le support, en une peptidyl thiohydantoïne (TH) C-terminale, qui comprend :
la mise en oeuvre d'un support solide fonctionnalisé par des groupes carboxyle,
la réaction du support solide avec un composé d'isoxazolium dans des conditions basiques, pour former des énol esters activés des groupes carboxyle, et
la réaction du support avec un isothiocyanate (ITC) choisi parmi l'ensemble comprenant l'ITC de trialkylsilyle et l'ITC de pyridine, pour former un anhydride mixte d'acide isothiocyanique et d'un acide carboxylique sur le support.

17. Procédé selon la revendication 16, dans lequel ledit ITC est l'ITC de triméthyle.

18. Procédé selon la revendication 16, dans lequel le composé d'isoxazolium est le sulfonate de 2-éthyl-5'-phénylisoxazolium.
